# EUROPEAN PATENT APPLICATION

(11) **EP 3 181 175 A1**
(43) Date of publication of application: **21.06.2017**
(21) Application number: 15839493.2
(22) Date of filing: 11.09.2015
(51) Int. Cl.: A61M 16/16

(54) **HUMIDIFICATION DEVICE**

(30) Priority: 11.09.2014 JP 2014184869
(71) Applicant: Metran Co., Ltd., Kawaguchi-shi, Saitama 332-0015 (JP)
(72) Inventor: OSADA Yasuo, Kawaguchi-shi Saitama 332-0015 (JP); NITTA Kazufuku, Kawaguchi-shi Saitama 332-0015 (JP); NITTA Dan, Kawaguchi-shi Saitama 332-0015 (JP); TOMOZAWA Satoshi, Kawaguchi-shi Saitama 332-0015 (JP)
(74) Representative: TBK
(86) International application number: PCT/JP2015/075860
(87) International publication number: WO 2016/039447

(57) **Abstract**

A humidifying device (nebulizer) includes a liquid receptor configured to temporarily store a liquid, a liquid transfer mechanism configured to transfer a liquid from a container to the liquid receptor, a receptor-side aerosol forming member configured to suck the liquid of the liquid receptor from a liquid suction port by a negative pressure produced by a gas jetted from a gas jetting part of a nozzle member, and form an aerosol of the liquid with a receptor-side aerosol generation nozzle, and a heating mechanism configured to heat at least part of the liquid of the liquid receptor or the liquid in the receptor-side aerosol forming member. A humidifying device that can perform efficient and hygienic heating by using a heater during humidification can thus be provided.

## Description

### Technical Field

The present invention relates to a humidifying device, and more particularly to a humidifying device for humidifying a gas such as air and oxygen supplied to a patient.

### Background Art

Supplying oxygen to a patient has conventionally been practiced in hospitals and the like. Oxygen generated by an oxygen cylinder and the like is supplied to a patient by using a mask and the like. The oxygen supplied from an oxygen cylinder and the like contains little moisture. In supplying the oxygen to an airway such as the nasal cavity of the patient, drying of the airway therefore needs to be prevented. A humidifying device is arranged on the way of the oxygen supply tube, so that humidified oxygen is supplied.

Among commonly known humidifying devices used to humidify oxygen is a nebulizer. This humidifying device is configured to include: a water bottle (container) which contains a liquid such as a medicine-dissolved solution, sterile water, purified water, distilled water, a physiological saline solution, and the like; a dedicated humidifying device adaptor (nebulizer adaptor) which is connected to the water bottle; and the like. The nebulizer adaptor is configured to jet out an oxygen gas from an orifice formed in a nozzle member, thereby sucking up the sterile water or the like contained in the water bottle from a suction hole arranged near the orifice and sucking air, and forming a fine aerosol of the sucked sterile water or the like to humidify a gas containing a high concentration of oxygen so that the humidified gas can be supplied to a patient.

If the water bottle of the sterile water or the like becomes empty, the water bottle needs to be replaced. Many nebulizer adaptors are therefore configured so that the water bottle can be replaced. A conventional nebulizer is configured to include a water supply pipe for sucking up the sterile water or the like from the water bottle to the nebulizer adaptor, and a drain tube for returning water accumulated in the nebulizer adaptor to the water bottle (see, for example, Japanese Patent Application Laid-Open No. 2012-071011).

The humidifying device may further employ a configuration in which a heater device is interposed between the water bottle (container) and the nebulizer adaptor to heat the sterile water or the like while sucking up the sterile water or the like contained in the water bottle, and the heated sterile water or the like is used to humidify a gas having a high oxygen concentration and supply the humidified gas to the patient (for example, see Japanese Patent No. 5485214).

### Summary of Invention

### Technical Problem

The heater device is configured so that the sterile water or the like passes through the heater device in direct contact with the interior of the heater device. There has thus been a problem that the heater device or its parts need to be sterilized each time another patient uses the heater device.

Conventionally, the sterile water or the like heated by the heater device and sucked up from the water bottle to the nebulizer adaptor is formed into an aerosol to humidify the oxygen gas and supplied to the patient with the oxygen gas. However, the sucked sterile water or the like does not all become the aerosol, but resides in part inside the nebulizer adaptor in the form of droplets. The sterile water or the like residing in the nebulizer adaptor therefore has needed to be returned by using the drain tube or the like. As a result, there has been a problem that germs are more likely to grow in the heater device or the water bottle.

In particular, the sterile water or the like sucked up from the water bottle to the nebulizer adaptor becomes an aerosol and mixed with the air taken in from inside the room. Here, germs in the room can also get in. Non-aerosol components of the sterile water or the like mixed with the germs included in the room air reside inside the nebulizer adaptor in the form of droplets. Furthermore, the sterile water or the like heated by the heater device resides in the nebulizer adaptor with temperatures that facilitate growth of the germs. Since such sterile water or the like accumulated in the nebulizer adaptor is let into the water bottle as a drain, the germs in the sterile water or the like enter the water bottle. The water bottle originally contains a liquid with little germs in it, like sterile water. Such water bottles are commercially available. However, there has been a problem that if the sterile water or the like returns to the water bottle as a drain, germs can get into the liquid, such as sterile water, in the water bottle.

Depending on conditions such as the state of the patient and the amount of oxygen supply, the sterile water or the like in the water bottle is consumed quickly, and the water bottle has needed to be frequently replaced with a new one. The nebulizer adaptor or the heater device is thus configured so that the water bottle is separated for replacement. In replacing the water bottle in the presence of the foregoing drain tube, the drain tube has needed to be reconnected from the old water bottle to the new water bottle. Since droplets from the nebulizer adaptor flow constantly through the drain tube as described above, there has been a problem that the water drips from the drain tube when the drain tube is reconnected from the old water bottle to the new water bottle.

The present invention has been achieved in view of the foregoing problems, and an object thereof is to provide a humidifying device that can perform efficient and hygienic heating by using a heater during humidification.

### Solution to Problem

To solve the foregoing problems, a humidifying device according to the present invention includes: a liquid receptor configured to temporarily store a liquid; a liquid transfer mechanism configured to transfer a liquid for humidification from a container containing the liquid to the liquid receptor; a receptor-side aerosol forming member configured to suck the liquid of the liquid receptor from a liquid suction port by a negative pressure produced by a gas jetted from a gas jetting part of a nozzle member, and form an aerosol of the sucked liquid with a receptor-side aerosol forming nozzle; and a heating mechanism configured to heat at least part of the liquid of the liquid receptor or the liquid in the receptor-side aerosol forming member.

As means associated with the foregoing humidifying device, the heating mechanism is arranged around the liquid receptor or the receptor-side aerosol forming member and configured to indirectly heat the liquid of the liquid receptor or the liquid in the receptor-side aerosol forming member from outside the member.

As means associated with the foregoing humidifying device, the heating mechanism is detachably arranged on the liquid receptor or the receptor-side aerosol forming member.

As means associated with the foregoing humidifying device, a connection portion configured to connect to the container is formed under the liquid receptor, and the heating mechanism is arranged on the liquid receptor or the receptor-side aerosol forming member so that the connection portion is exposed.

As means associated with the foregoing humidifying device, the heating mechanism includes a pair of heater members that open and close horizontally, and the heater members are arranged to sandwich the liquid receptor from both side surfaces.

As means associated with the foregoing humidifying device, the receptor-side aerosol forming member has the liquid suction port in a position horizontally shifted from a position vertically directly below the receptor-side aerosol forming nozzle.

As means associated with the foregoing humidifying device, the liquid suction port of the receptor-side aerosol forming member is arranged near a liquid surface of the liquid of the liquid receptor.

As means associated with the foregoing humidifying device, a restriction member configured to restrict impingement of the gas jetted from the gas jetting part of the nozzle member on the liquid surface is arranged.

As means associated with the foregoing humidifying device, the liquid receptor includes a partial storage portion configured to section a part of a storage space storing the liquid, the heating mechanism heats at least the liquid of the partial storage portion, and the liquid suction port of the receptor-side aerosol forming member is arranged to suck the liquid of the partial storage portion.

As means associated with the humidifying device, a partition wall is erected in the storage space of the liquid receptor, and the partial storage portion and the remaining portion are sectioned by the partition wall.

As means associated with the humidifying device, the liquid receptor includes a communication passage configured to connect the partial storage portion and the remaining portion of the storage space.

As means associated with the humidifying device, an opening of the communication passage on a side of the remaining portion is arranged in a position higher than an opening on a side of the partial storage portion.

As means associated with the humidifying device, a slope is formed on at least a part of a bottom surface of the liquid receptor.

As means associated with the humidifying device, the liquid transfer mechanism includes a transfer channel configured to suck the liquid for humidification from the container containing the liquid and discharge the liquid to the liquid receptor by using a negative pressure produced by the gas jetted from the gas jetting part of the nozzle member.

As means associated with the humidifying device, a discharge port of the liquid of the transfer channel includes a container-side aerosol forming nozzle configured to form an aerosol of the liquid sucked from the container.

As means associated with the humidifying device, a cutoff member configured to move between a cutoff position in which to cut off the discharge port of the liquid of the transfer channel from the gas jetting part and a retracted position in which to be retracted from the cutoff position, a floating member configured to float up and down according to a rise or fall of the liquid surface of the liquid stored in the liquid receptor, and a connection member configured to connect the floating member to the cutoff member and cause the cutoff member to move with floating of the floating member are provided.

As means associated with the humidifying device, a determination device configured to determine lowering of the liquid surface of the liquid of the liquid receptor, and notification means configured to issue a notification that the container containing the liquid becomes empty on the basis of a result of determination of the determination device are provided.

As means associated with the humidifying device, a heater temperature sensor configured to measure a temperature of a heater configured to heat the liquid with the heating mechanism is provided, and the determination device determines the lowering of the liquid surface of the liquid in the liquid receptor on the basis of a measurement value of the heater temperature sensor.

As means associated with the humidifying device, the determination device determines that the liquid surface of the liquid in the liquid receptor has lowered if an output of the heater is not increased and the temperature of the heater increases.

As means associated with the humidifying device, a gas temperature sensor configured to measure a temperature of a gas humidified by the aerosol is further provided, and the determination device determines that the liquid surface of the liquid in the liquid receptor has lowered if the temperature of the humidified gas deviates from a set temperature and the temperature of the heater increases.

As means associated with the humidifying device, an adaptor that is detachably attached to the container is provided, and the adaptor includes at least the receptor-side aerosol forming member, the liquid receptor, and the liquid transfer mechanism.

As means associated with the humidifying device, the humidifying device is configured to humidify and send the gas containing oxygen to a patient, and includes a sending unit configured to send out a mixture of the gas and the aerosol.

As means associated with the humidifying device, the container is provided.

### Advantageous Effects of Invention

According to the foregoing humidifying device of the present invention, an excellent effect that sterile water or the like can be efficiently and hygienically heated to warm the humidified gas during use can be provided.

### Brief Description of Drawings

FIG. 1 is a cross-sectional view for describing a configuration of a nebulizer according to a first embodiment of the present invention.
FIGs. 2(A) to 2(D) are cross-sectional views for describing an attachment and detachment mechanism of a nebulizer adaptor and a water bottle.
FIG. 3 is a side view for describing an air suction port formed by a window of an adjustment dial and a window of an erected projection portion.
FIGs. 4(A) to 4(C) are cross-sectional views and bottom views showing examples of a positional relationship between an ejection port of a liquid of a container-side aerosol forming member and an ejection port of a liquid of a receptor-side aerosol forming member.
FIG. 5 is a cross-sectional view showing a distance from an orifice of a nozzle member in a jetting direction of an oxygen gas and a graph showing as a general concept a relationship between the distance and a negative pressure at the part of an ejection port of a liquid.
FIG. 6 is a side view and a bottom view showing a distance from a center of the orifice of the nozzle member, and a graph showing as a general concept a relationship between the distance and the negative pressure at the part of the ejection port of a liquid.
FIG. 7 is a side view showing an example of an oxygen flowmeter.
FIG. 8(A) is a perspective view showing a heater device, and FIG. 8(B) is a front view showing an operation panel of the heater device.
FIG. 9(A) is a plan view showing the heater device and the nebulizer adaptor, and FIG. 9(B) is a cross-sectional view showing a state in which a gas temperature sensor of the heater device enters the horizontal projection portion of the nebulizer adaptor.
FIG. 10 is a plan view showing the heater device and the nebulizer adaptor with a swing case opened.
FIGs. 11(A) and 11(B) are both plan views showing an internal structure of plate heaters arranged in the swing case.
FIGs. 12(A) to 12(F) are conceptual diagrams showing an internal structure and operation of a lock mechanism of the heater device.
FIG. 13 is a block diagram showing a control configuration of the heater device.
FIG. 14 is a flowchart showing a basic control of the heater device.
FIG. 15 is a flowchart showing a heater control of the heater device.
FIG. 16 is an enlarged cross-sectional view showing a liquid surface control mechanism of the nebulizer adaptor.
FIG. 17 is a perspective view showing a positional relationship between a discharge port of a transfer channel of the container-side aerosol forming member, the ejection portion of the receptor-side aerosol forming member, and an orifice of the nozzle member.
FIG. 18 is an enlarged view for describing a structure of a valve of the liquid surface control mechanism of the nebulizer adaptor.
FIG. 19 is an enlarged perspective view of a part of the valve.
FIG. 20(A) is a cross-sectional view for describing a configuration of a nebulizer according to a second embodiment of the present invention, and FIG. 20(B) is a cross-sectional view taken along the line B-B of FIG. 20(A).
FIG. 21(A) is a cross-sectional view for describing an application example of the nebulizer, and FIG. 21(B) is a cross-sectional view taken along the line B-B of FIG. 21(A).
FIG. 22(A) is a cross-sectional view for describing a configuration of a nebulizer according to a third embodiment of the present invention, and FIG. 22(B) is a cross-sectional view taken along the line B-B of FIG. 22(A).
FIG. 23(A) is a cross-sectional view for describing an application example of the nebulizer, and FIG. 23(B) is a cross-sectional view taken along the line B-B of FIG. 23(A).
FIG. 24(A) is a cross-sectional view for describing an application example of the nebulizer, and FIG. 24(B) is a cross-sectional view taken along the line B-B of FIG. 24(A).
FIG. 25(A) is a cross-sectional view for describing an application example of the nebulizer, and FIG. 25(B) is a cross-sectional view taken along the line B-B of FIG. 25(A).
FIG. 26 is a plan view of a heater device for describing an application example of the nebulizer.
FIG. 27(A) is a conceptual diagram for describing a structure of a valve of a liquid surface control mechanism included in a nebulizer adaptor that constitutes a nebulizer according to a fourth embodiment of the present invention, and FIG. 27(B) is an enlarged view showing essential parts of the structure shown in FIG. 27(A).
FIG. 28(A) is a conceptual diagram for describing a structure of a valve of a liquid surface control mechanism included in a nebulizer adaptor that constitutes a nebulizer according to a fifth embodiment of the present invention, and FIG. 28(B) is a top view showing essential parts of the structure shown in FIG. 28(A).
FIGs. 29(A) and 29(B) are conceptual diagrams for describing a structure of a liquid surface control mechanism included in a nebulizer adaptor that constitutes a nebulizer according to a sixth embodiment of the present invention.
FIGs. 30(A) and 30(B) are conceptual diagrams for describing a structure of a liquid surface control mechanism included in a nebulizer adaptor that constitutes a nebulizer according to a seventh embodiment of the present invention.
FIG. 31 is a cross-sectional view for describing a configuration of a nebulizer according to an eighth embodiment of the present invention.
FIG. 32 is a flowchart showing an application example in which status determination processing of the nebulizer adaptor is added to the control configuration of the nebulizer according to the first embodiment of the present invention.
FIG. 33(A) is a plan view showing a state in which a heat transfer measurement temperature sensor intended for temperature correction of the gas temperature sensor is arranged on the nebulizer according to the first embodiment of the present invention, and FIG. 33(B) is a cross-sectional view showing a state in which the gas temperature sensor of the heater device enters the horizontal projection portion of the nebulizer adaptor.
FIG. 34(A) is a front view and a sectional side view showing an application example of the lock mechanism of the nebulizer according to the first embodiment of the present invention in an unlockable state and before an unlocking operation of an operator, and FIG. 34(B) is a front view and a sectional side view in the unlockable state and after the unlocking operation of the operator.
FIG. 35(A) is a front view and a sectional side view showing an application example of the lock mechanism in a non-unlockable state and before an unlocking operation of the operator, and FIG. 35(B) is a front view and a sectional side view in the non-unlockable state and after the unlocking operation of the operator.
FIG. 36(A) is a cross-sectional view showing a contracted state of a locking plunger 89 of the lock mechanism, and FIG. 36(B) is a cross-sectional view showing an extended state thereof.
FIG. 37 is a chart showing control patterns of the locking plunger 89 of the lock mechanism.

### Description of Embodiments

Nebulizers according to respective embodiments of the present invention will be described below with reference to the drawings.

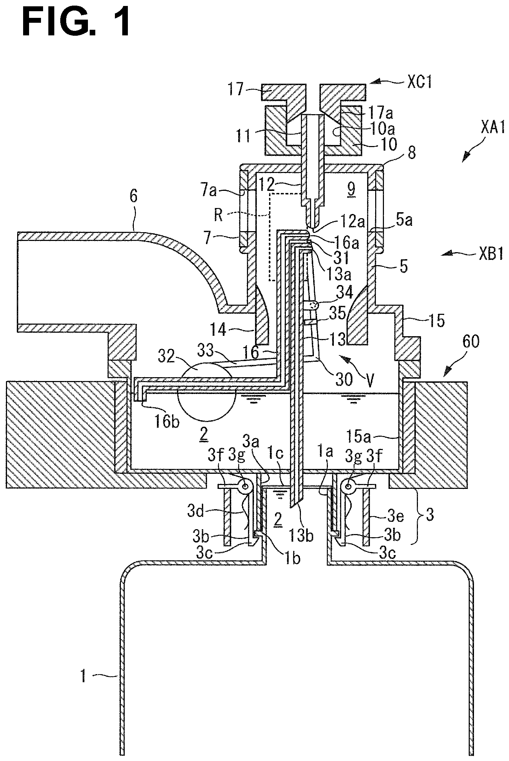

[First Embodiment] Initially, a nebulizer XA1 according to a first embodiment will be described with reference to FIGs. 1 to 15. In these diagrams and subsequent diagrams, some components are omitted as appropriate for simplification.

The nebulizer (humidifying device) XA1 illustrated in FIG. 1 includes a nebulizer adaptor XB1, a water bottle (container) 1, and a heater device (heating mechanism) 60. The water bottle 1 is filled with a liquid 2 such as sterile water. An opening portion 1a is arranged on the top of the water bottle 1. The opening portion 1a is covered with a film 1c before use. A pipe on a side of the nebulizer adaptor XB1 is configured to pierce through and break the film 1c when in use. The hole formed in the film 1 by the pipe is configured to shrink in diameter into close contact with the pipe by itself. A container-side connection portion 1b intended for coupling with the nebulizer adaptor XB1 is arranged on an outer peripheral surface of the opening portion 1a. The container-side connection portion 1b is a ring-shaped engagement projection extending radially outward. The container-side connection portion 1b is engaged and coupled with a counterpart. While the water bottle 1 is shown to be completely filled with the liquid 2, air may also be contained.

An adaptor-side connection portion 3 intended for coupling with the foregoing water bottle 1 is arranged on a lower part of the nebulizer adaptor XB1. The adaptor-side connection portion 3a includes a cylindrical portion 3a which surrounds the opening portion 1a, a pair of engagement arms 3b which are arranged outside the cylindrical portion 3a, spring members 3d which bias end projections 3c of the engagement arms 3b toward the outer periphery of the cylindrical portion 3a, and a ring member 3e configured to operate the engagement arms 3b. A lower end of the cylindrical portion 3a makes contact with the container-side connection portion 1b for mutual positioning. The engagement arms 3b are configured to be swingable about swing shafts 3g. The engagement arms 3b swing radially with respect to the cylindrical portion 3a so that the projections 3c at the ends (lower ends) are engaged with the container-side connection portion 1b. The ring member 3e is configured to, when lifted up, make contact with operation levers 3f of the engagement arms 3b. The ring member 3e can thereby swing the engagement arms 3b about the swing shafts 3g in directions of disengaging the lower end projections 3c from the container-side connection portion 1b against the force of the spring members 3d, so that the engagement arms 3b are forcefully opened.

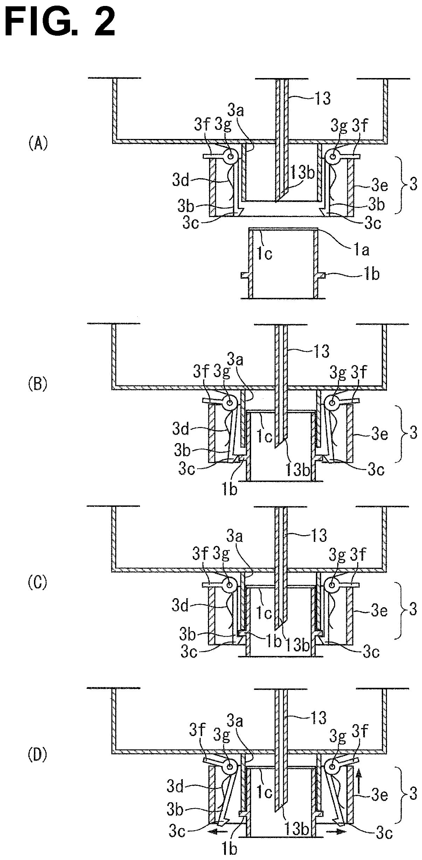

To connect the adaptor-side connection portion 3 and the container-side connection portion 1b, as shown in FIGs. 2(A) and 2(B), the two portions are brought close to each other and the lower end projections 3c of the engagement arms 3b are brought into contact with the container-side connection portion 1b with the lower end of a transfer channel 13b driven through the film 1c. The lower end projections 3c of the engagement arms 3b have a tapered surface. The lower end projections 3c of the engagement arms 3b are automatically pushed apart by the container-side connection portion 1b, and the lower end projections 3c and the container-side connection portion 1b are axially engaged and coupled with each other as shown in FIG. 2(C). To separate the adaptor-side connection portion 3 and the container-side connection portion 1b, as shown in FIG. 2(D), the ring member 3e can be lifted up to disengage the lower end projections 3c of the engagement arms 3b from the container-side connection portion 1b. According to the present structure, the connection and disconnection can be easily performed by one operation with significantly improved workability. It will be appreciated that the adaptor-side connection portion 3 and the container-side connection portion 1b may be coupled by a screw structure as heretofore. Although not shown in the diagrams, a gasket may be provided inside the adaptor-side connection portion 3. When the container-side connection portion 1b and the adaptor-side connection portion 3 are coupled to each other, leakage of liquid from the coupling part can be suppressed by the action of the gasket.

Return to FIG. 1. An erected projection portion 5 of cylindrical shape is formed on the nebulizer adaptor XB1 in a direction (perpendicular direction) that becomes vertical when the nebulizer XA1 is erected. This erected projection portion 5 constitutes a supply system of an oxygen gas. A horizontal projection portion 6 of cylindrical shape is formed on a side of the nebulizer adaptor XB1 away from the erected projection portion 5, in a direction (horizontal direction) that becomes lateral when the nebulizer XA1 is erected. The nebulizer adaptor XB1 is configured so that a mixture of air, the oxygen gas, and an aerosol can be sent out to a patient from the horizontal projection portion 6. In other words, the horizontal projection portion 6 serves as a mixture discharge system.

A rotatable adjustment dial 7 is arranged on the outside of the erected projection portion 5. The top of the erected projection portion 5 is closed by a top plate 8. A closed space 9 constituted by the projection portion 5 and the top plate 8 is thus formed inside the erected projection portion 5.

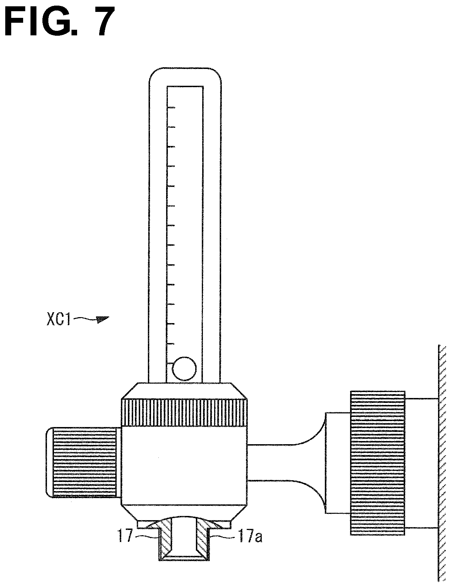

A terminal 11 equipped with a nut 10 is fitted into the top plate 8. The nut 10 is connected to an outlet member 17 of an oxygen flowmeter XC1 such as shown in FIG. 7, whereby the oxygen gas is supplied.

More specifically, a female screw portion 10a arranged on the nut 10 and a male screw portion 17a arranged on the outlet member 17 of the oxygen flowmeter XC1 are threadedly engaged to bring the outlet portion 17 of the oxygen flowmeter XC1 and the terminal 11 into close contact with each other. A flow hole formed in the outlet member 17 of the oxygen flowmeter XC1 and a flow hole formed in the terminal 11 communicate with each other to supply the oxygen gas from the oxygen flowmeter XC1 to the terminal 11.

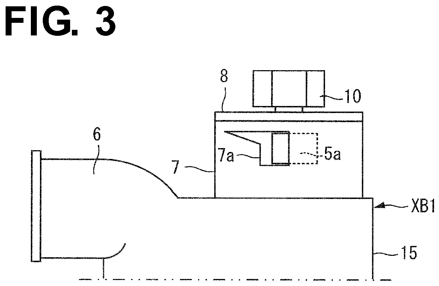

As shown enlarged in FIG. 3, windows 7a are formed in a side surface of the adjustment dial 7. Windows 5a are formed in a side surface of the erected projection portion 5 in positions opposed to the windows 7a. Openings formed by the windows 7a and the windows 5a have a function as an air suction hole.

If the adjustment dial 7 is rotated to oppose the windows 7a to the windows 5a, the openings communicating with the closed space 9 are formed. In other words, the rotation position of the adjustment dial 7 can be adjusted to adjust the area of the openings to the closed space 9, whereby the amount of intake air can be adjusted.

Return to FIG. 1, a description will be given. A nozzle-like diffuser 14 is arranged inside the erected projection portion 5 of cylindrical shape. This diffuser 14 is not shaped to spread out in the bottom portion but formed in a tapered shape with a spreading upper end portion, and the portion below the tapered portion is formed in a straight pipe shape. A nozzle member 12 is arranged above the tapered portion of the diffuser 14.

With the nozzle member 12 and the diffuser 14 arranged as described above, the oxygen gas jetted from an orifice 12a of the nozzle member 12 passes through the diffuser 14 at high speed. The air residing in the closed space 9 is thus sucked to flow toward the diffuser 14. Here, air is sucked according to the area of the openings formed by the windows 7a and the windows 5a corresponding to the rotation position of the adjustment dial 7, and passes through the diffuser 14.

The orifice 12a (gas jetting part) is formed in the end of the nozzle member 12. An ejection port (aerosol forming nozzle) 16a of a receptor-side aerosol forming member 16 and an ejection port (aerosol forming nozzle) 13a of a container-side aerosol forming member 13 are arranged near the orifice 12a of the nozzle member 12. The container-side aerosol forming member 13 also has a function as a mechanism (liquid transfer mechanism) for transferring the liquid for humidification (such as sterile water) from the water bottle 1 to a liquid receptor 15a of the nebulizer adaptor XB1.

Since the nozzle member 12, the receptor-side aerosol forming member 16, and the container-side aerosol forming member 13 have respective different functions, the members are configured as respective independent members. The three members are configured to be combined with each other.

However, the positional relationship between the oxygen gas jetted from the orifice 12a of the nozzle member 12 and the ejection ports 16a and 13a for ejecting the liquid 2 is subtle and difficult to adjust. As shown by dotted lines R, at least two members arbitrarily selected from among the nozzle member 12, the receptor-side aerosol forming member 16, and the container-side aerosol forming member 13, or preferably the three members, are desirably integrally configured with each other.

The container-side aerosol forming member 13 is arranged inside the diffuser 14 which is arranged in a housing 15 of the nebulizer adaptor XB1. The ejection port 13a of the liquid 2 is formed near the orifice 12a of the nozzle member 12. The transfer channel 13b for sucking up the liquid 2 is formed continuously with the ejection port 13a. The lower end of the transfer channel 13b extends to inside the water bottle 1 and inserted into the water 2 such as sterile water, so that the liquid 2 such as sterile water can be efficiently sucked up.

The liquid receptor 15a is arranged on the lower part of the housing 15 of the nebulizer adaptor XB1. Part of the liquid 2 ejected from the ejection port 13a of the container-side aerosol forming member 13 is formed into an aerosol by a negative pressure produced by the oxygen gas jetted from the orifice 12a of the nozzle member 12, and humidifies the oxygen gas. A mixture of the air, the oxygen gas, and the aerosol is sent out from the horizontal projection portion 6 toward the patient. Meanwhile, part of the liquid 2 ejected from the ejection port 13a forms droplets and drops into the liquid receptor 15a, whereby the liquid 2 is temporarily accumulated. In particular, the ejection port 13a of the container-side aerosol forming member 13 is located in a position farther from the orifice 12a in the oxygen jetting direction than the ejection port 16a of the receptor-side aerosol forming member 16 is, and the amount of the sucked liquid 2 that drops down is adjusted to be greater than that becomes an aerosol. In other words, the main object of the receptor-side aerosol forming member 13 is to suck up and transfer the liquid 2 in the water bottle 1 to the liquid receptor 15a of the housing 15 of the nebulizer adaptor XB1.

In the present embodiment, the water bottle 1 is configured to shrink with a negative pressure in the bottle. As the air and the liquid 2 in the water bottle 1 are sucked up via the transfer channel 13b, the water bottle 1 shrinks so that unnecessary air or liquid will not flow back into the water bottle 1. This suppresses intrusion of germs into the liquid 2 in the water bottle 1 and eliminates the need to extend the lower end of the transfer channel 13b to the bottom of the water bottle 1. It will be understood that if the water bottle 1 has a non-shrinking structure, the lower end of the transfer channel 13b is desirably extended to near the bottom of the bottle.

The receptor-side aerosol forming member 16 is formed inside the diffuser 14 which is arranged in the housing 15 of the nebulizer adaptor XB1. The ejection port 16a of the receptor-side aerosol forming member 16 is formed near the orifice 12a of the nozzle member 12. The lower end of the receptor-side aerosol forming member 16 extends to the liquid receptor 15a. A liquid suction port 16b is arranged near the liquid surface at a prescribed water level. This liquid suction port 16b is located in a position horizontally shifted from a position vertically directly below the ejection port 16a. For example, the liquid suction port 16b is arranged in a position 5 mm or more above, preferably 10 mm or more above, the bottom surface of the liquid receptor 15a.

The liquid 2 accumulated in the liquid receptor 15a is sucked rom the liquid suction port 16b and ejected as an aerosol from the ejection port 16a of the liquid 2 of the receptor-side aerosol forming member 16. The resulting mixture of the air, the oxygen gas, and the aerosol is sent out from the horizontal projection portion 6 to the patient. Here, the liquid 2 ejected as the aerosol from the foregoing container-side aerosol forming member 13 is also mixed in.

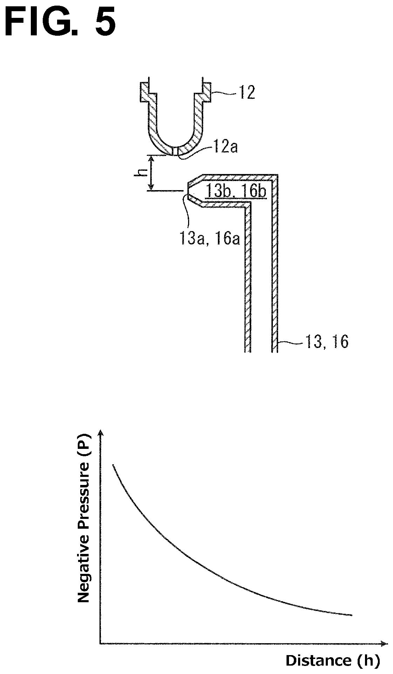

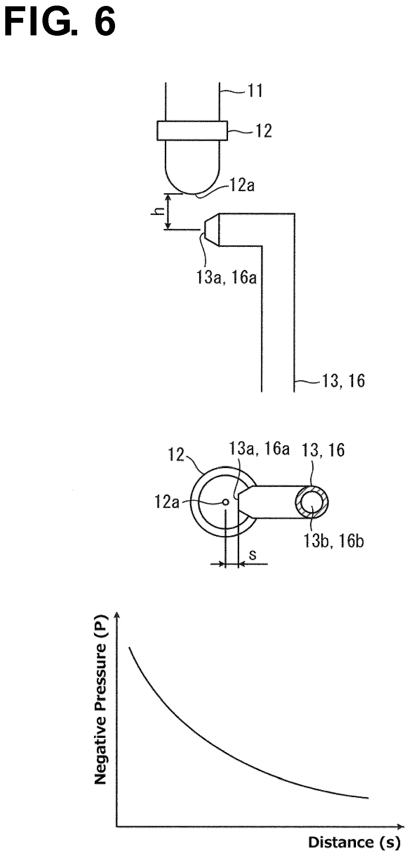

The positional relationship between the ejection port 13a of the container-side aerosol forming member 13 and the ejection port 16a of the receptor-side aerosol forming member 16 is set so that if a cutoff member 31 of a liquid surface control mechanism V to be described later is located in a retracted position, the amount of the liquid 2 transferred to the liquid receptor 15a by the container-side aerosol forming member 13 exceeds the consumption of the liquid 2 discharged outside by the receptor-side aerosol forming member 16 and the container-side aerosol forming member 13. An example is shown in FIG. 1, where the ejection port 13a of the container-side aerosol forming member 13 is arranged in a position farther from the orifice 12a in the oxygen jetting direction (see the reference sign h in FIG. 5) than the ejection port 16a of the receptor-side aerosol forming member 16 is. In addition, the ejection port 13a of the container-side aerosol forming member 13 is arranged closer in a radial direction of the oxygen jetting (see the reference sign s in FIG. 6) than the ejection port 16a of the receptor-side aerosol forming member 16 is. Such an arrangement makes the negative pressure occurring in the ejection port 13a of the container-side aerosol forming member 13 greater than the negative pressure occurring in the ejection port 16a of the receptor-side aerosol forming member 16, whereby the force for sucking up the liquid 2 in the water bottle 1 can be secured.

The container-side aerosol forming member 13 is configured so that the lower end of the transfer channel 13b for sucking up the liquid 2, the transfer channel 13b being connected to the ejection port 13a, extends to the water bottle 1. The function of the highest priority of the container-side aerosol forming member 13 is to suck up the liquid 2 from the lower part of the water bottle 1. It will be understood that the container-side aerosol forming member 13 may at the same time form the ejected liquid 2 into an aerosol to humidify the oxygen gas so that the mixture of the air, the oxygen gas, and the aerosol is sent out from the horizontal projection portion 6 to the patient.

On the other hand, the role or the function of the highest priority of the receptor-side aerosol forming member 16 is to eject the liquid 2 that is drawn up by the container-side aerosol forming member 13 and accumulated in the liquid receptor 15a, out of the ejection port 16a of the receptor-side aerosol forming member 16 in the form of aerosol. The receptor-side aerosol forming member 16 is thus extended to below the prescribed water level of the liquid receptor 15a and near the water surface. The distance from the liquid suction port 16b arranged at the lower end of the receptor-side aerosol forming member 16 to the ejection port 16a of the liquid 2 is small, and the negative pressure needed of the receptor-side aerosol forming member 16 to suck up the liquid 2 is thus small.

The reason why the liquid suction port 16b of the receptor-side aerosol forming member 16 is located not near the bottom surface of the liquid receptor 15a but near the water surface is to suck heated water by priority, because high-temperature water heated by the heater device 60 to be described later rises to the water surface side. The reason why the liquid suction port 16b is located in a position horizontally shifted from the position vertically directly below the ejection port 16a is that the water surface vertically directly below the ejection port 16a tends to ripple because of the oxygen from the orifice 12a, and low-temperature water on the bottom side of the liquid receptor 15a and the high-temperature water on the water surface side are likely to be mixed up. In other words, the liquid suction port 16b is horizontally shifted to effectively suck only a high-temperature liquid 2 in a region where the water surface is calm.

The ejection port 16a of water of the receptor-side aerosol forming member 16 is arranged in a position where the negative pressure is relatively low in the graphs of the negative pressure shown in FIG. 5 and 6. The ejection port 13a of the liquid 2 of the container-side aerosol forming member 13 is arranged in a position where the negative pressure is relatively high. Such an arrangement thus makes sense.

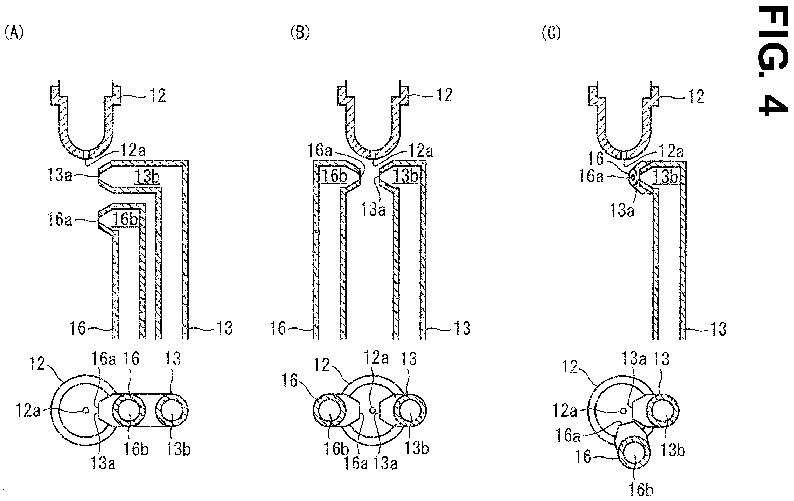

FIG. 1 illustrates, as an example, a case where the ejection port 16a of the reception-side aerosol forming member 16 and the ejection port 13a of the container side aerosol forming member 13 are shown to be vertically separated from each other and arranged to eject the liquid 2 in the same direction. However, the present invention is not limited thereto. For example, as shown in FIG. 4(A), the ejection port 16a of the receptor-side aerosol forming member 16 may be arranged in a position farther from the orifice 12a in the oxygen jetting direction than the ejection port 13a of the container-side aerosol forming member 13 is. In such a case, the ejection port 16a of the receptor-side aerosol forming member 16 and the ejection port 13a of the container-side aerosol forming member 13 may preferably be arranged in substantially the same positions in the radial direction of the oxygen jetting. As shown in FIG. 4(B), the ejection port 16a of the receptor-side aerosol forming member 16 may be arranged in a position opposed to the ejection port 13a of the container-side aerosol forming member 13. In such a case, the ejection port 16a of the receptor-side aerosol forming member 16 may preferably be arranged in a position farther than the ejection port 13a of the container-side aerosol forming member 13 is in the radial direction of the oxygen jetting. As shown in FIG. 4(C), the ejection port 13a of the container-side aerosol forming member 13 and the ejection port 16a of the receptor-side aerosol forming member 16 may be arranged at the same height position but in positions rotated by a certain angle about the center of the orifice 12a of the nozzle member 12 when seen from below. In such a case, the ejection port 16a of the receptor-side aerosol forming member 16 may preferably be arranged in a position farther than the ejection port 13a of the container-side aerosol forming member 13 is in the radial direction of the oxygen jetting. It will be understood that these three examples are not restrictive. Other configurations may be employed as long as the ejection port 13a of the liquid 2 of the container-side aerosol forming member 13 and the ejection port 16a of the liquid 2 of the receptor-side aerosol forming member 16 are arranged to satisfy the condition about the negative pressures needed.

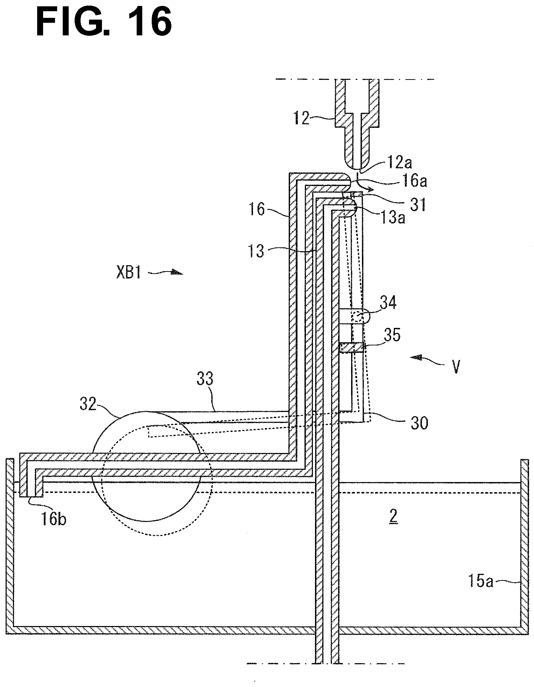

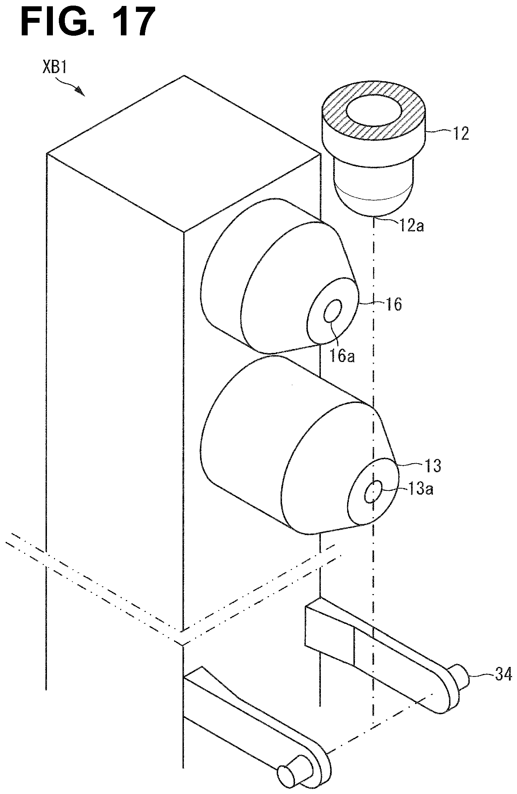

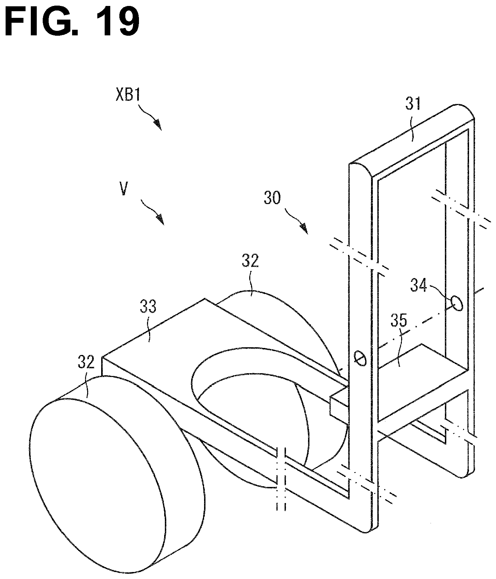

As shown in FIGs. 16 to 19, a liquid surface control mechanism V is arranged in the nebulizer adaptor XB1. The liquid surface control mechanism V has a function of maintaining the liquid 2 stored in the liquid receptor 15a at a prescribed water level. Specifically, if the liquid 2 of the liquid receptor 15a reaches the prescribed water level, the suction capability of the container-side aerosol forming member 13 is reduced. On the other hand, if the liquid 2 of the liquid receptor 15a falls below the prescribed water level, the suction capability of the container-side aerosol forming member 13 is increased.

Details will be described. The liquid surface control mechanism V includes a valve 30 which makes an opening and closing operation vertically above the ejection port 13a and thereby prevents the oxygen jetted from the orifice 12a from impinging on the ejection port 13a of the container-side aerosol forming member 13. The valve 30 includes a cutoff member 31, floating members 32, a connection member 33, and a stopper 35.

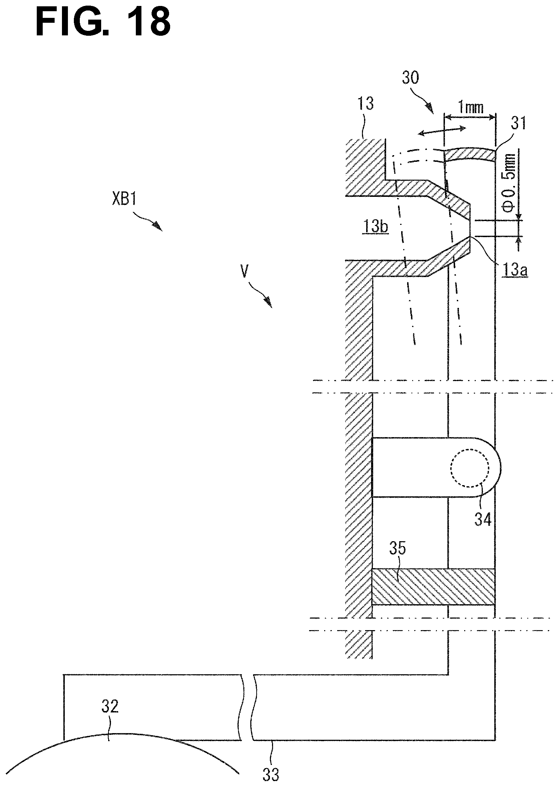

The cutoff member 31 swings about a swing shaft 34 between a cutoff position (position shown by solid lines in FIG. 18) in which to cut off the ejection port 13a of the container-side aerosol forming member 13 from the orifice 12a of the nozzle 12 and a retracted position (position shown by dashed-dotted lines in FIG. 18) in which to be retracted from the cutoff position. If the cutoff member 31 is located in the cutoff position, the cutoff member 31 cuts off the ejection port 13a of the container-side aerosol forming member 13 from the orifice 12a of the nozzle member 12 to stop the function of sucking up the liquid 2 by the container-side aerosol forming member 13. If the cutoff member 31 is located in the retracted position, the cutoff member 31 cancels the cutting off of the ejection port 13a of the container-side aerosol forming member 13 from the orifice 12a in the nozzle member 12 to restore the function of sucking up the liquid 2 by the container-side aerosol forming member 13.

The floating members 32 float on the liquid surface of the liquid 2 stored in the liquid receptor 15a, and floats up and down according to a rise or fall of the liquid surface. The connection member 33 is a member that connects the floating member 32 and the cutoff member 31. The connection member 33 is bent halfway in an L shape. The swing shaft 34 is arranged in the middle of the connection member. The floating of the floating members 32 causes rotational moment on the swing shaft 34, so that the cutoff member 31 swings.

As shown in FIG. 18, the stopper 35 is arranged on the connection member 33. If the cutoff member 31 swings in a cutoff direction, the stopper 35 comes into contact with a side surface of the container-side aerosol forming member 13 to position the cutoff member 31. If the cutoff member 31 swings in a retracting direction, the cutoff member 31 itself comes into contact with the container-side aerosol forming member 13 to position itself.

Consequently, in the nebulizer adaptor XB1, as the container-side aerosol forming member 13 continues sucking up the liquid 2 in the water bottle and the liquid surface of the liquid 2 stored in the liquid receptor 15a rises, the floating members 32 float up and the cutoff member 31 swings to the cutoff position. This stops the function of sucking up the liquid 2 by the container-side aerosol forming member 13. The liquid 2 accumulated in the liquid receptor 15a is thereby prevented from overflowing into the horizontal projection portion 6 (see FIG. 1). As the receptor-side aerosol forming member 16 sucks up the liquid 2 stored in the liquid receptor 15a and the liquid surface of the liquid 2 falls, the floating members 32 float down and the cutoff member 31 swings to the retracted position. This restores the function of sucking up the liquid 2 by the container-side aerosol forming member 13. In the present embodiment, the cutoff position of the cutoff member 31 is such that a virtual line connecting the cutoff member 31 and the swing shaft 34 is parallel to or coincident with the jetting direction of oxygen from the orifice 12a. In such a position, when the oxygen from the orifice 12a is received, the pressure of the oxygen gas is radially received by the swing shaft 34 via the cutoff member 31. Rotational moment is thus less likely to occur. The result is that the pressure of the oxygen gas is unlikely to have an adverse effect on the rotation of the cutoff member 31 resulting from the buoyancy of the floating members 32 according to a change in the liquid level of the liquid 2.

Next, the heater device 60 will be described. The heater device 60 heats the liquid 2 of the liquid receptor 15a or the liquid 2 in the receptor-side aerosol forming member 16 from outside the housing 15 of the nebulizer adaptor XB1.

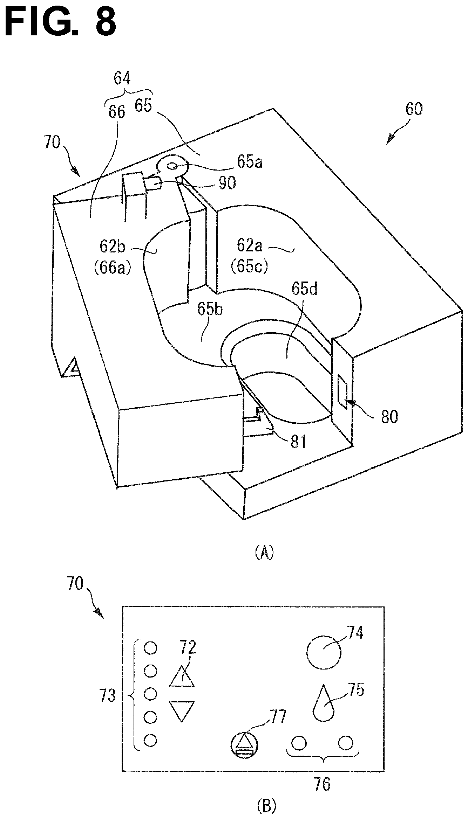

As shown in FIG. 8(A), the heater device 60 includes a pair of plate heaters 62a and 62b which is arranged to surround the side surface of the housing 15 of the liquid receptor 15a, a case 64 which accommodates the plate heaters 62a and 62b, an operation panel 70 which is formed on a side surface of the case 64, and a lock mechanism 80. The case 64 includes a main case 65 and a swing case 66 which is horizontally swingably arranged on the main case 65 via a hinge 65a.

The main case 65 includes a bottom surface 65b which holds the lower side of the nebulizer adaptor XB1, and an inner peripheral surface 65c which holds a half of the side surface of the liquid receptor 15a. The plate heater 62a is arranged in the inner peripheral surface 65c. Although not shown in the diagram, the main case 65 accommodates a power supply device, a controller (control device), and the like. An opening 65d is formed in the bottom surface 65b of the main case 65 so that the adaptor-side connection portion 3 of the nebulizer adaptor XB1 can be exposed below. That is, the space of this opening 65d can be used to directly connect the nebulizer adaptor XB1 and the water bottle 1 (see FIG. 1). The swing case 66 includes an inner peripheral surface 66a which holds a half of the side surface of the liquid receptor 15a. The plate heater 62b is arranged in the inner peripheral surface 66a.

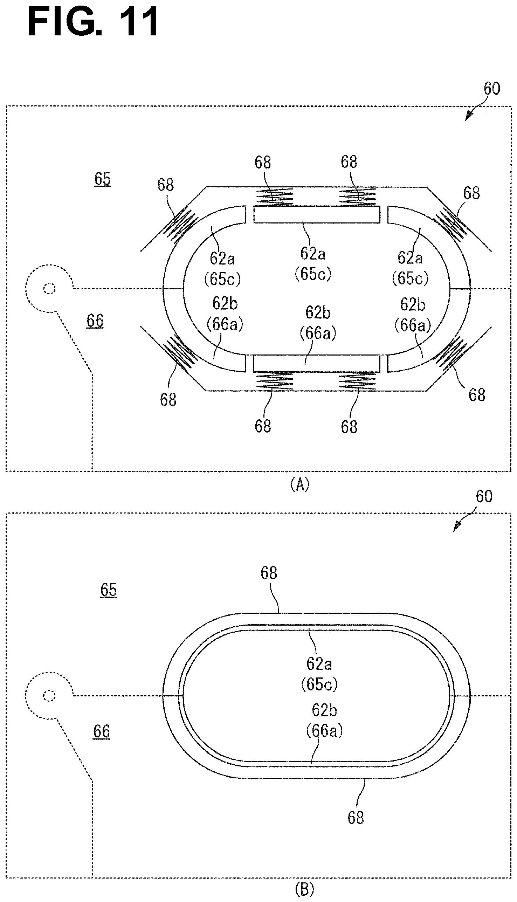

As shown in FIG. 11(A), the plate heaters 62a and 62b are provided with elastic members 68 which are arranged on surfaces (rear surfaces) opposite from the surfaces opposed to the housing 15 of the nebulizer adaptor XB1. Examples of the elastic members 68 may include a spring. The elastic members 68 press the plate heaters 62a and 62b against the housing 15 for close contact. As shown in FIG. 11(B), heat insulating cushion materials may be arranged as the elastic members 68 on the rear sides of the plate heaters 62a and 62b.

With such a structure, this heater device 60 can horizontally open and close the pair of plate heaters 62a and 62b. The pair of plate heaters 62a and 62b thus sandwiches the liquid receptor 15a from both sides. The plate heaters 62a and 62b and the liquid receptor 15a are brought into close contact by the pressing force of the elastic members 68.

As shown in FIG. 8(B), the operation panel 70 is formed on a side surface of the main case 65. This operation panel 70 includes a temperature setting section 72 configured to set the temperature of the mixture supplied from the nebulizer adaptor XB1, a set temperature display section 73 configured to display the temperature set by the temperature setting section 72, a heater switch 74, a no-water indication section 75 configured to indicate that the liquid receptor 15a is empty of water, a heater temperature display section 76 configured to display the temperatures of the plate heaters 62a and 62b, and an unlock button 77 configured to unlock the lock mechanism 80. The temperature setting section 72 includes up and down, a pair of buttons. The up button is pressed to increase the set temperature. The down button is pressed to decrease the set temperature. The set temperature display section 72 includes five lamps which are directly arranged. The lamps indicate the set temperature in five levels, 28 degrees, 31 degrees, 34 degrees, 37 degrees, and 40 degrees, respectively. The heater switch 74 switches ON/OFF the plate heaters 62a and 62b. The heater temperature display section 76 includes two color lamps. A blue lamp is lit if the actual temperature of the plate heaters 62 and 62b is lower than or equal to 30 degrees. A red lamp is lit if the actual temperature exceeds 30 degrees. This heater temperature indication section 76 can be checked to see whether the plate heaters 62a and 62b are at safe-to-touch temperatures. In other words, the heater temperature display section 76 displays whether the swing case 66 can be opened.

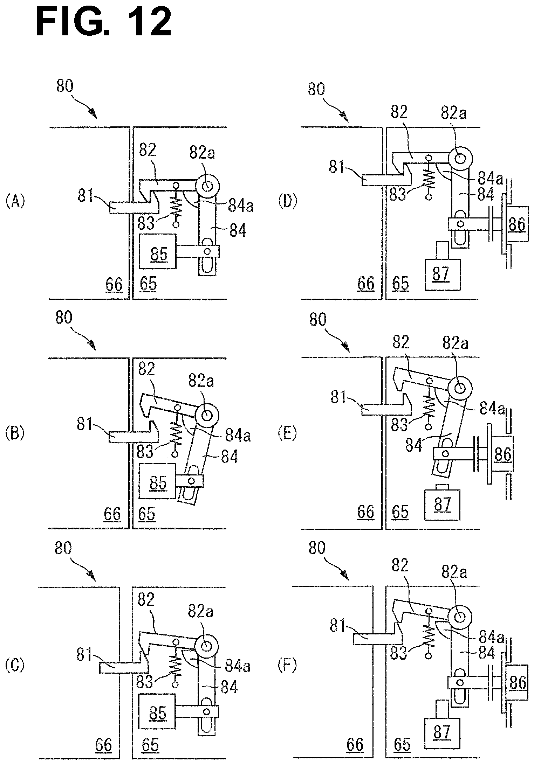

As shown in FIG. 12(A), the lock mechanism 80 includes a fixed engagement portion 81 which is arranged on a side of the swing case 66, a swing engagement portion 82 which is arranged on a side of the main case 65, a locking spring 83 configured to bias the swing engagement portion 82 to a lock side, an operation arm 84 configured to move with the swing engagement portion 82 only in a direction of opening the swing engagement portion 82, and a driving member 85 configured to electrically forcefully swing the swing engagement portion 82 via the operation arm 84. The ends of the fixed engagement portion 81 and the swing engagement portion 82 are engaged with each other to maintain the swing case 66 and the main cage 65 in a closed state. The swing engagement portion 82 is arranged to be capable of swinging about a swing shaft 82a. The operation arm 84 is arranged to be capable of swinging about the swing shaft 82a, and includes a pressing portion 84a which makes contact with the swing engagement portion 82. Examples of the driving member 85 may include an electromagnetic plunger. The driving member 85 is connected to the operation arm 84 which moves with the swing portion 82, and forcefully swings the operation arm 84.

If the release button 77 shown in FIG. 8(B) is pressed, the driving member 85 swings the operation arm 84 to an open side as shown in FIG. 12(B). As a result, the pressing portion 84a makes contact with the swing engagement portion 82 to swing the swing engagement portion 82 to the open side against the biasing force of the locking spring 83. Thus, the swing engagement portion 82 is disengaged from the fixed engagement portion 81 to unlock the lock mechanism 80. The swing case 66 can thus be opened. If the plate heaters 62a and 62b exceed a predetermined temperature (when the red lamp of the heater temperature display section 76 is lit), the driving member 85 will not unlock the lock mechanism 80 even when the release button 77 is pressed. The reason is to prevent the operator from touching the plate heaters 62a and 62b and getting burned.

On the other hand, in closing the swing case 66, the swing case 66 is manually pressed against the main case 65. As shown in FIG. 12(C), the fixed engagement portion 81 and the swing engagement portion 82 are thereby pressed against each other. The two portions interfere with each other, and if the fixed engaged portion 81 is presses against the swing engagement portion 82, the swing engagement portion 82 can swing against the biasing force of the locking spring 83 to enter a locking state of FIG. 12(A) by itself.

As shown in FIGs. 12(D), 12(E), and 12(F), the driving member 85 may be replaced with an opening/closing button 86 which can be operated to manually swing the operation arm 84 for opening and closing. In such a case, if the plate heaters 62a and 62b exceed the predetermined temperature, a locking plunger 89 is preferably engaged with the operation arm 84 to restrict the swing of the operation arm 84 to the open side.

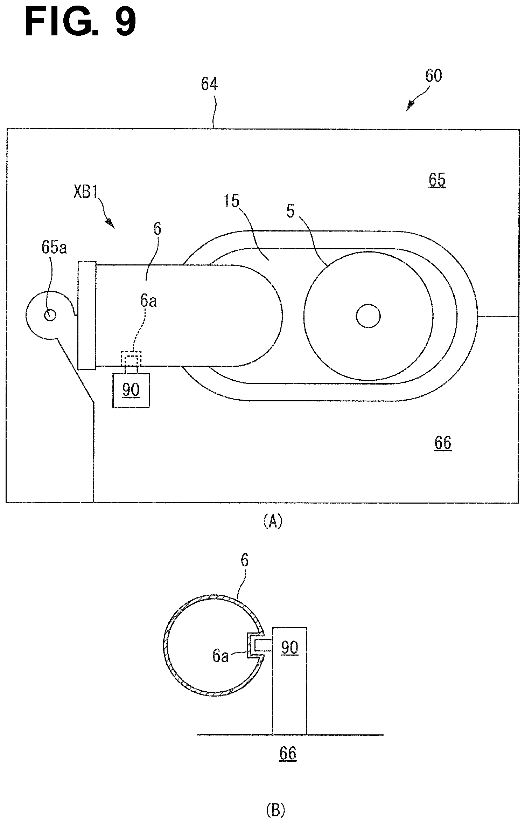

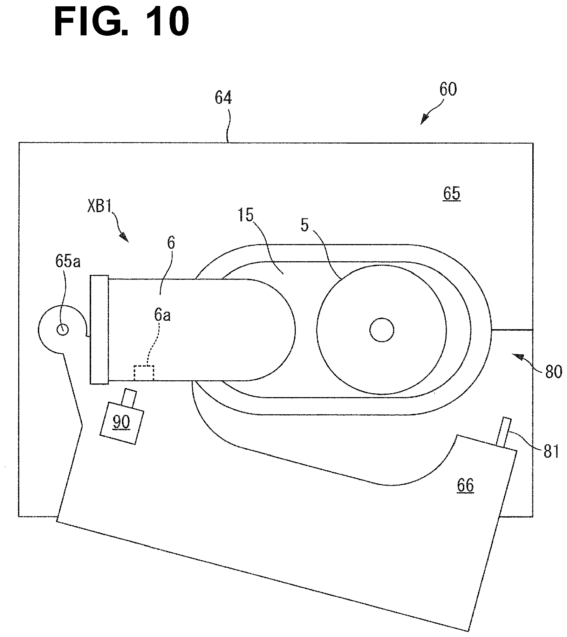

As shown in FIGs. 8 to 10, a gas temperature sensor 90 is arranged on the top surface of the swing case 66. This gas temperature sensor 90 swings with the swing case 66, and, as shown in FIG. 9(B), is accommodated in a sensor accommodation recess 6a formed in the outer peripheral surface of the horizontal projection portion 6 of the nebulizer adaptor XB1. The sensor accommodation recess 6a is recessed toward the center of the horizontal projection portion 6, so that the gas temperature sensor 90 can approach the center of the horizontal projection portion 6. This makes the member temperature of the sensor accommodation recess 6a close to the temperature of the supplied mixed gas. The gas temperature sensor 90 thus measures the temperature of the sensor accommodation recess 6a to indirectly measure the temperature of the mixed gas. The controller corrects the temperature measured by the gas temperature sensor 90 for measurement errors occurring from the interposition of the housing (sensor accommodation recess 6a) to determine the temperature of the mixed gas. Since the gas temperature sensor 90 is not in direct contact with the mixed gas, the gas temperature sensor 90 does not need to be replaced or sterilized for each patient. As shown in FIG. 10, the gas temperature sensor 90 moves to and from the sensor accommodation recess 6a according to the swing operation of the swing case 66. The gas temperature sensor 90 therefore stays out of the way when the swing case 66 is opened to separate the nebulizer adaptor XB1 and the heater device 60.

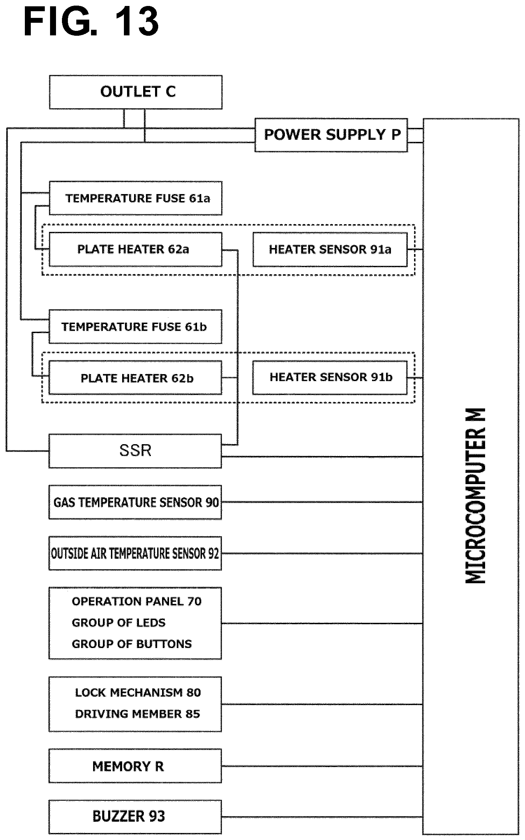

FIG. 13 shows a control configuration of the heater device 60. This control configuration has a function of controlling the temperature of the humidified gas to a set temperature, and at the same time serves as a no-water determination device configured to determine the presence or absence of the liquid 2 in the liquid receptor 15a to indirectly determine whether the water bottle 1 runs out of water. This heater device 60 is controlled by a microcomputer M and a solid state relay SSR. Aside from the gas temperature sensor 90, the microcomputer M is connected with heater (temperature) sensors 91a and 91b which are installed on the pair of plate heaters 62a and 62b, an outside air temperature sensor 92 configured to measure the ambient temperature, and a buzzer 93 configured to notify abnormality. The microcomputer M is further connected with a power supply P which is connected to an outlet C, a group of LEDs and a group of setting buttons of the operation panel 70, the driving member (plunger) 85 of the lock mechanism 80, a memory R, the solid state relay SSR, and the like. The power supply P converts a 100-V power supply from the outlet C into 5 V and supplies the resultant to the microcomputer M.

One of the terminals of the outlet C is connected to the plate heaters 62a and 62b via temperature fuses 61a and 61b in series. The other terminal of the outlet C is connected to the plate heaters 62a and 62b via the solid state relay SSR. If the plate heaters 62a and 62b are abnormally heated to reach or exceed a preset temperature due to a trouble of the heater (temperature) sensors 91a and 91b, the SSR, the microcomputer M, or the like, the temperature fuses 61a and 61b blow by themselves to stop the power supply to the plate heaters 62a and 62b.

The solid state relay SSR controls the power supplied to the plate heaters 62a and 62b on the basis of a PWM signal from the microcomputer M. The microcomputer M issues the PWM signal to the solid state relay SSR so that the gas temperature sensor 90 stabilizes at the set temperature set by the operation panel 70. Aside from the control of the plate heaters 62a and 62b, the values obtained from the heater (temperature) sensors 91a and 91b are used for purposes such as to monitor abnormal heating. The value obtained from the outside air temperature sensor 92 is used to further correct the correction amount of the gas temperature sensor 90.

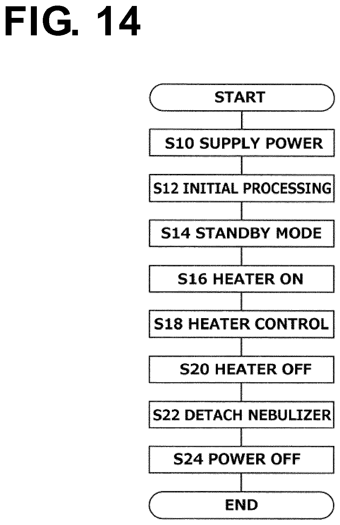

FIG. 14 shows a basic control flow of the heater device 60. In step S10, power is supplied from the outlet C. In step S12, respective variables are initialized for initial processing. Proceeding to step S14, a heater output is maintained at 0 for a standby mode. Next, in step S16, heater heating is turned ON from the operation panel 70. After an operation check on the respective temperature sensors, the processing proceeds to step S18 to perform heater control. Then, in step S20, the heater heating is turned OFF, and the heater output is set to 0. Proceeding to step S22, nebulizer detachment processing is performed. In step S24, the power supply to the outlet C is turned off, and the processing ends.

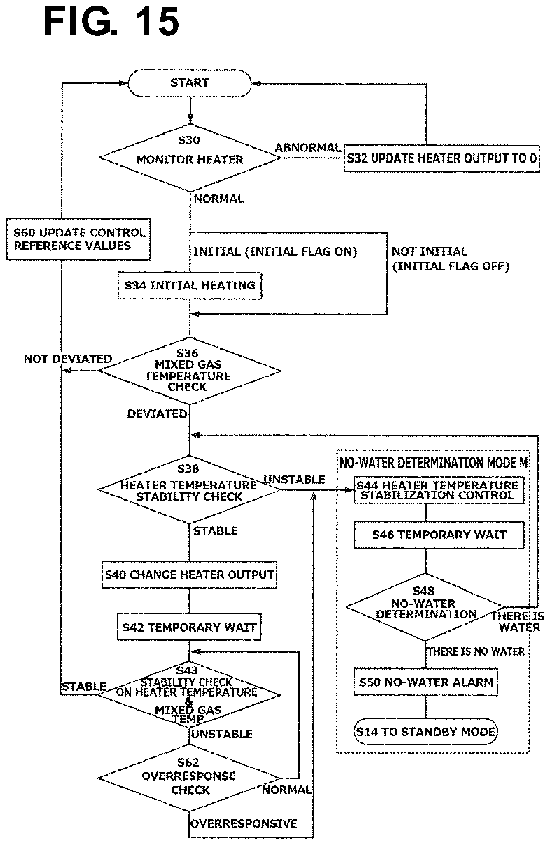

FIG. 15 shows a detailed flow which is repeated at regular periods (for example, 10 seconds) in the heater control of step S18. Initially, in step S30, a heater state is monitored to check for a temperature abnormality, heater disconnection, and unattachment of the nebulizer adaptor XB1 (using a not-shown mechanical switch). If the heater state is abnormal, then in step S32, the heater output is updated to 0 and the processing returns to step S30. On the other hand, if, in step S30, the heater state is normal, the processing proceeds to step S34 to perform initial heating processing.

The initial heating processing is processing to be performed only when the output of the plate heaters 62a and 62b is switched from OFF to ON (processing to be performed only when an initial flag is ON). The output of the plate heaters 62a and 62b is initially increased stepwise. The plate heaters 62a and 62b are then set to a first output, and after a certain time of wait for temperature stabilization, the heater temperature (first heater temperature), the temperature of the mixed gas (first gas temperature), and the ambient temperature (first ambient temperature) are measured. The plate heaters 62a and 62b are then set to a second output, and after another wait for temperature stabilization, the heater temperature (second heater temperature), the temperature of the mixed gas (second gas temperature), and the ambient temperature (second ambient temperature) are measured. From differences between the various temperatures at the first output and those at the second output, the amount of change in the temperature of the mixed gas per unit amount of change in output (or the amount of change in output needed to cause a unit amount of change in the gas temperature) is calculated as a first control reference value. The amount of change in the temperature of the heater per unit amount of change in output (or the amount of change in output for a unit amount of change in the heater temperature) is calculated as a second control reference value. The initial flag is then turned OFF. Subsequently, by using the first control reference value, the plate heaters 62a and 62b are controlled so that the temperature of the mixed gas actually discharged from the nebulizer XA1 becomes the set temperature.

Specifically, preset fixed values may be used as the first and second outputs. For example, the output value of the plate heaters 62a and 62b when the temperature of the mixed gas is settled at a first temperature target value by feedback control and the like may be used as the first output. The output value of the plate heaters 62a and 62b when the temperature of the mixed gas is settled at a second temperature target value may be used as the second output. For example, the output value of the plate heaters 62a and 62b when the temperature of the mixed gas is settled at the first temperature target value (for example, set temperature) by feedback control and the like may be used as the first output. A value obtained by adding or subtracting a fixed amount to/from the first output may be used as the second output. In any case, the foregoing first and second control reference values can be calculated by detecting values at which the temperature of the mixed gas stabilizes under at least two respective conditions in which the mixed gas has different temperatures.

Next, in step S36, a mixed gas temperature check is performed. The mixed gas temperature check is processing for determining whether a difference between the temperature of the mixed gas measured by the gas temperature sensor 90 and the set temperature falls within predetermined thresholds (for example, ±0.5 degrees) (stable) or not (unstable). If the difference falls within the predetermined thresholds, the processing returns to step S30 since the current control may be continued. In step S36, the heater temperature and the heater output are recorded in the memory R. The heater temperature and the heater output are used as initial values for control in a no-water determination mode to be described later and in recovering from a no-water state (i.e., at a time of recovery with the initial flag OFF).

On the other hand, if the difference between the temperature of the mixed gas and the set temperature exceeds the predetermined thresholds, the processing proceeds to step S38 to perform a heater temperature stability check. The heater temperature stability check is performed by evaluating the degree of change in the heater temperature of the plate heaters 62a and 62b in a predetermined time. For example, the heater temperature is measured in units of one minute. If the amount of change between the previous temperature and the current temperature falls within a predetermined threshold, the heater temperature is determined to be stable. If the amount of change exceeds the predetermined threshold, the heater temperature is determined to be unstable. Alternatively, a value obtained by differentiating the amount of change may be used as the determination value. A moving average value may be used as the determination value.

If the heater temperature is stable, the processing proceeds to heater output change processing of step S40, in which the output of the plate heaters 62a and 62b is changed to eliminate the difference between the temperature of the mixed gas and the set temperature. This changed output value is determined by calculating the amount of change in output needed to eliminate the temperature difference between the temperature of the mixed gas and the set temperature by using the already-calculated first control reference value, and adding or subtracting the amount of change in output to/from the current output. If the calculation result exceeds a heater temperature upper limit value or/and a heater output upper limit value which are set in advance, a maximum output within the range of the upper limit value(s) is determined as the changed output value. It will be understood that the changed output value is the same as the current output value if there is no difference between the temperature of the mixed gas and the set temperature.

Next, after temporary wait processing of step S42 to wait for a certain time (for example, 20 seconds) until the temperature of the plate heaters 62a and 62b and the temperature of the mixed gas change after the change in output, the processing proceeds to step S43 to perform a stability check on the heater temperature and the mixed gas temperature. If both the heater temperature and the mixed gas temperature are stable, the processing proceeds to step S60 to update the foregoing first and second control reference values by using the amount of change in the temperature of the mixed gas and the amount of change in the temperature of the heater before and after the change in output. The latest mixed gas temperature and heater temperature are both stored into the memory R, and the processing returns to step S30. In such a manner, optimum control reference values for a change in the external environment (change in temperature or humidity) or a change in flowrate can be constantly retained.

On the other hand, if, in step S43, either of the heater temperature and the mixed gas temperature is determined to be unstable, the processing proceeds to step S62 to perform an overresponse check. Specifically, if the output of the plate heaters 62a and 62b is increased, then whether the temperature of the mixed gas exceeds the set temperature by more than a predetermined amount (for example, +0.5 degrees) is checked. If the output of the plate heaters 62a and 62b is decreased, then whether the temperature of the mixed gas falls below the set temperature by more than a predetermined amount (for example, - 0.5 degrees) is checked. The reason is that if the mixed gas temperature is determined to be overresponsive to the output control of the plate heaters 62a and 62b, the water may have run out. In step S62, if the mixed gas temperature is determined to be overresponsive, the processing proceeds to step S44 to enter a no-water determination mode M. On the other hand, if, in step S62, the mixed gas temperature is determined to not be overresponsive, the stability check of the heater temperature and the mixed gas temperature in step S43 is repeated again.

The no-water determination mode M will be described. If the heater temperature is unstable in step S38, or if the mixed gas temperature is overresponsive in step S62, the processing proceeds to heater temperature stabilization control processing of step S44, in which output control is performed to stabilize the temperature of the heater and a consecutive number of times counter is incremented by one. Specifically, the output value of the plate heaters 62a and 62b is changed on the basis of the second control reference value so that the current heater temperature becomes a stabilization target temperature, with the heater temperature recorded in the memory R immediately before in step S36 or the like as the stabilization target temperature. Then, after temporary wait processing of step S46 to wait for a certain time (for example, several minutes) until the temperature of the plate heaters 62a and 62b stabilizes after the change in output, a no-water determination is performed in step S48. The no-water determination of step S48 is performed depending on the number of the consecutive number of times counter of step S44. In the present embodiment, if the consecutive number of times counter is less than or equal to six, it is determined that there still is water, and the processing returns to step S38 to perform the heater temperature stability check. If the heater temperature is not stable, the heater temperature stabilization control processing of step S44 is repeated.

If the heater temperature does not converge at the stabilization target value in the heater temperature stability check of step S38 even after the heater temperature stabilization control of step S44 is repeated six times, then the consecutive number of times counter of step S48 reaches seven. As a result, it is determined that an abnormal change has occurred in the external environment of the heater, i.e., the water has run out, and no-water alarm processing is performed in step S50. In the no-water alarm processing, the output of the plate heaters 62a and 62b is updated to 0. In addition, the no-water indication section 75 of the operation panel 70 is lit and the buzzer 93 issues an alarm to appeal to both the auditory and visual senses of the user. The processing then returns to step S14 of the basic control flow for standby.

As described above, in the present embodiment, both the determination as to the amount of deviation of the temperature of the mixed gas from the target value (set value) and the determination as to the unstable level of the heater temperature are evaluated. If the mixed gas temperature deviates from the target value and the heater temperature is stable, it is determined that the set temperature is changed or the flowrate of the mixed gas has changed. The heater output is then controlled so that the mixed gas temperature follows the target value. On the other hand, if the heater temperature is unstable and the heater temperature remains unstable even after the heater output is controlled with the heater temperature itself as the target value, then the no-water determination is performed. In particular, in the present embodiment, the processing enters the no-water determination mode M only if the mixed gas temperature deviates from the target value. This enables accurate, efficient no-water determination. In the present embodiment, the no-water determination is described to be made automatically in the process of controlling the heater output. However, the present invention is not limited thereto. For example, if the heater output is maintained constant (fixed), the temperature of the mixed gas and the heater temperature are constantly monitored. If the temperature of the mixed gas and the heater temperature continue to increase for a certain period, the water is determined to have run out. If the temperature of the mixed gas and the heater temperature increase temporarily and then both the temperatures stabilize, the water is determined to not have run out, because the variations are caused by a change in the flowrate of the mixed gas. In other words, a temporary change in the heater temperature may be allowed while a continuous temperature increase over a certain period may be considered to be caused by running out of water and thus the water can be determined to have run out.

In the nebulizer XA1 described above, the container-side aerosol forming member 13 sucks the liquid 2 for humidification from the water bottle 1 containing the liquid 2 and stores the liquid 2 in the liquid receptor 15a by using the negative pressure produced by the oxygen gas jetted from the orifice 12a of the nozzle member 12. By using the same negative pressure, the receptor-side aerosol forming member 16 sucks the liquid 2 from the liquid receptor 15a and forms an aerosol of the liquid 2. A drain tube for returning the liquid 2, such as sterile water, accumulated in the liquid receptor 15a to the container, such as the water bottom 1, therefore does not need to be provided. This facilitates the replacement of the container, such as the water bottle 1, and solves the conventional problem that the liquid 2 drips from the drain tube when the container is replaced.

In the nebulizer XA1, the liquid 2, such as sterile water, to be a drain containing germs in the room will not return to the container, such as the water bottle 1. The drawback that the germs can get into the liquid in the container can thus be solved.

In the nebulizer XA1, the liquid 2 stored in the liquid receptor 15a is heated by the heater device 60 and formed into an aerosol by the receptor-side aerosol forming member 16. As compared to the conventional case where the liquid 2 is rapidly heated in the process of sucking up the liquid 2 from the water bottle 1, the stored liquid 2 can be stably heated. This allows a reduction in power consumption. After heated in the liquid receptor 15a, part of the liquid 2 sucked up and ejected by the receptor-side aerosol forming member 16 returns to the liquid receptor 15a. This is extremely hygienic because the liquid 2 is preferentially reused as an aerosol without flowing back to the water bottle (container) 1 side. Moreover, since the liquid suction port 16b of the receptor-side aerosol forming member 16 is arranged not near the bottom surface of the liquid receptor 15a but near the prescribed water level, high-temperature water which tends to collect to the water surface can be sucked by priority. Since the liquid suction port 16b is horizontally shifted from the position vertically directly below the ejection port 16a, only high-temperature water can be efficiently sucked from the calm water surface with little rippling.

Furthermore, this nebulizer XA1 can indirectly detect that the water bottle 1 runs out of water, by detecting the lowering of the liquid surface of the liquid receptor 15a. If the lowering of the liquid surface of the liquid receptor 15a is detected early, the liquid 2 in the liquid receptor 15a serves as a buffer when the water bottle 1 has completely run out of the liquid 2. The humidification function can thus be maintained while the water bottle 1 is replaced. In particular, in the present embodiment, if the water surface in the liquid receptor 15a lowers, the temperature of the plate heaters 62a and 62b increases continuously. Such a tendency is utilized to indirectly detect the lowering of the liquid surface in the liquid receptor 15a from temperature changes of the plate heaters 62a and 62b. Whether the water bottle 1 runs out of water can thus be accurately determined without preparing a special no-water detection device.

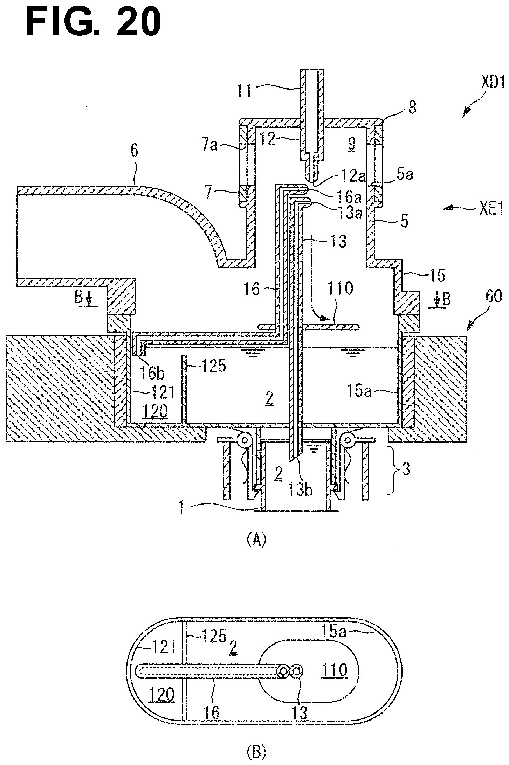

[Second Embodiment] Next, a configuration of a nebulizer XD1 according to a second embodiment will be described with reference to FIG. 20. Here, characteristic parts of the nebulizer XD1 will be mainly described. The same configuration and operation as those of the foregoing first embodiment will be denoted by the same reference numerals in the drawings, and a description thereof may be omitted as appropriate. To avoid complication of the drawings, a description and depiction of the liquid surface control mechanism are omitted.

This nebulizer (humidifying device) XD1 includes a nebulizer adaptor XE1, the heater device 60, and the water bottle (container) 1.

In this nebulizer adaptor XE1, a restriction member 110 configured to restrict impingement of the gas jetted from the orifice 12a of the nozzle member 12 on the water surface of the liquid receptor 15a is arranged vertically directly below the receptor-side aerosol forming nozzle 16a and above the water surface. This restriction member 110 is a horizontally-extending plate member fixed to the receptor-side aerosol forming member 16 or the contain-side aerosol forming member 13, and receives the gas jetted from the orifice 12a. This suppresses the rippling of the water surface of the liquid receptor 15a due to the jetting of the gas. This allows the warm water in the liquid receptor 15a heated by the heater device 60 to reside calmly near the water surface, so that only the stable warm water can be efficiently sucked from the liquid suction port 16b of the receptor-side aerosol forming member 16.

This nebulizer adaptor XE1 further includes a partial storage portion 120 which is formed by sectioning a part of the storage space for storing the liquid 2 in the liquid receptor 15a. More specifically, this partial storage portion 120 includes a partial peripheral wall 121 which encloses part of the liquid 2. In the present embodiment, a partition wall 125 is erected in the liquid receptor 15a. This partition wall 125 sections the storage space into the partial storage portion 120 and the remaining portion. Some kind of passage through which the liquid 2 can move is needed between the partial storage portion 120 and the remaining portion of the storage space. In the present embodiment, the height of the partition wall 125 is set to be lower than the prescribed water level, and the liquid 2 moves via the water surface. In particular, the volume of the partial storage portion 120 is set to be smaller than that of the remaining portion.

The heater device 60 heats at least the liquid 2 in the partial storage portion 120 from outside via the partial peripheral wall 121. The liquid suction port 16b of the receptor-side aerosol forming member 16 sucks the liquid 2 of the partial storage portion 120 by priority. This helps the liquid 2 heated in the partial storage portion 120 by the heater device 60 to reside in the partial storage portion 120. This can quickly increase the water temperature, so that the mixed gas can be efficiently heated. Although not shown in the diagram, the heater of the heater device 60 can be arranged in the partition wall 125 to heat the partition wall 125 so that the partial storage portion 120 can be heated more efficiently.

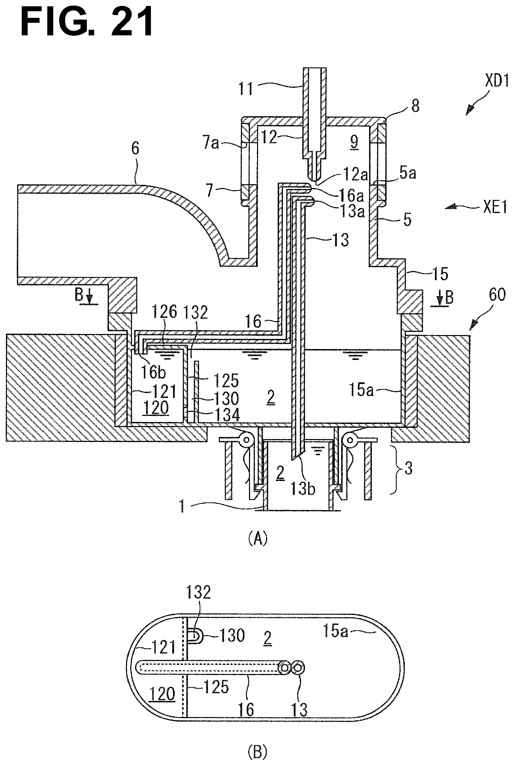

For example, as shown in an application example of FIG. 21, a lid member 126 may be arranged on the partial storage portion 120 sectioned by the partition wall 125 (or the height of the partition wall 125 may be set to be higher than the prescribed water level) to prevent the liquid 2 from entering from the upper side (water surface side) of the partial storage portion 120. A communication passage 130 configured to connect the partial storage portion 120 and the remaining portion of the storage space is arranged in the liquid receptor 15a. An opening 132 of the communication passage 130 on the remaining portion side is arranged above the bottom surface of the liquid receptor 15a. An opening 134 on a side of the partial storage portion 120 is arranged on the bottom surface side of the partial storage unit 120. As a result, the opening 132 of the communication passage 130 on the remaining portion side is located in a position higher than the opening 134 on the partial storage portion side. Consequently, the warm water heated in the partial storage portion 120 can rise and reside in the partial storage portion 120. The liquid suction port 16b of the receptor-side aerosol forming member 16 can thus preferentially suck only the heated warm water. If the water level in the partial storage portion 120 lowers, the liquid 2 in the remaining portion side moves to the partial storage portion 120 side through the communication passage 130. Since the opening 132 of the communication passage 130 on the remaining portion side is located near the prescribed water level, only warm water heated in the remaining portion side and residing at the water surface can be exclusively transferred to a side of a partial storage portion 120. Since the opening 134 of the communication passage 130 on the partial storage portion 120 side is located near the bottom surface of the partial storage portion 120, the liquid 2 moved to the partial storage portion 120 is heated by the heater device 60 to rise, and is sucked from the liquid suction port 16b of the receptor-side aerosol forming member 16. Like the present application example, if the structure is less susceptible to the rippling of the liquid surface, the restriction member may be omitted.

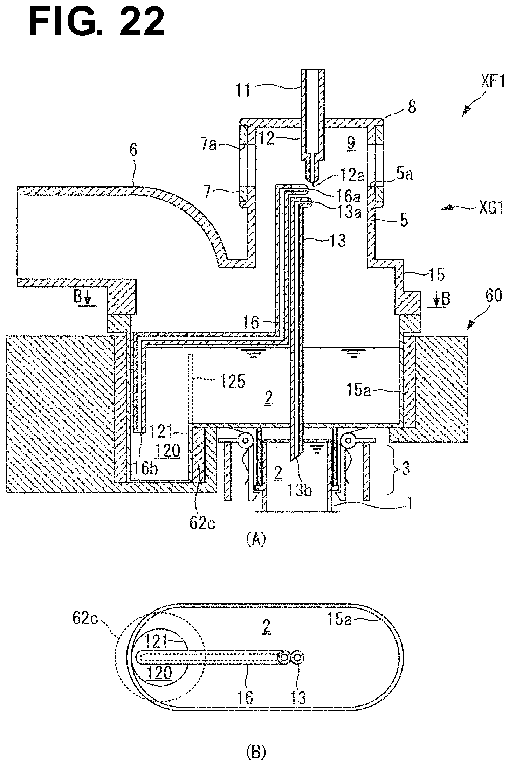

[Third Embodiment] Next, a configuration of a nebulizer XF1 according to a third embodiment will be described with reference to FIG. 22. Here, characteristic parts of the nebulizer XF1 will be mainly described. The same configuration and operation as those of the foregoing first and second embodiments will be denoted by the same reference numerals in the drawings, and a description thereof may be omitted as appropriate. To avoid complication of the drawings, a description and depiction of the liquid surface control mechanism are omitted.

This nebulizer (humidifying device) XF1 includes a nebulizer adaptor XG1, the heater device 60, the water bottle (container) 1.

In this nebulizer adaptor XG1, the bottom surface of the storage space for storing the liquid 2 in the liquid receptor 15a is partially lowered. This deep-bottomed portion constitutes the partial storage portion 120. In other words, this partial storage portion 120 is formed by sectioning a storage space of the liquid 2 in a height direction. The peripheral surface of the deep bottom of the partial storage portion 120 constitutes the partial peripheral wall 121. Some kind of passage through which the liquid 2 can move is needed between the partial storage portion 120 and the remaining portion of the storage space. In the present embodiment, the upper part of the partial storage portion 120 is open and the liquid 2 can move freely.

The heater device 60 includes a partial heater 62c which is arranged to surround the periphery or bottom surface of the partial storage portion 120. The liquid 2 in the partial storage portion 120 can thus be intensively heated. The liquid suction port 16b of the receptor-side aerosol forming member 16 is arranged near the partial storage portion 120 to preferentially suck the liquid 2 that is intensively heated in the partial storage unit 120. As shown by the dotted lines, a partition wall 125 like that of the second embodiment may be additionally provided between the partial storage portion 120 and the remaining portion. This partition wall 125 can further limit the movement of the liquid 2 between the partial storage portion 120 and the remaining portion.

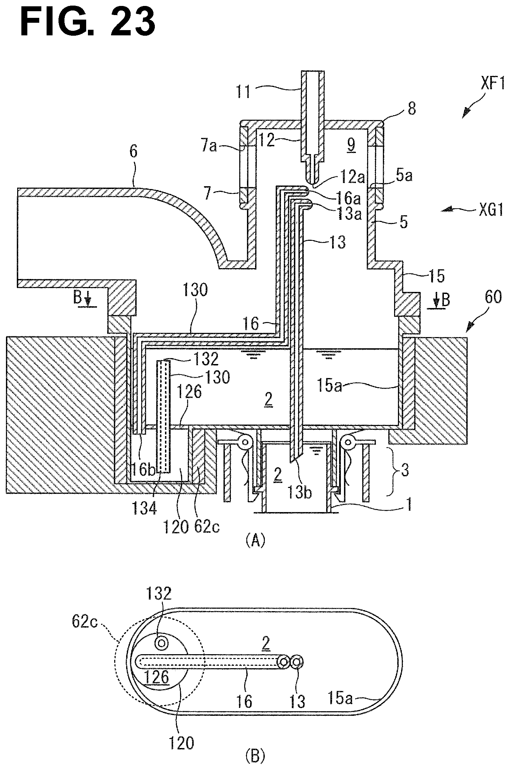

For example, as shown in an application example of FIG. 23, a lid member 126 may be arranged on the partial storage portion 120 sectioned by the deep-bottomed space so that the liquid 2 does not enter from the upper side (water surface side) of the partial storage portion 120. In such a case, a communication passage 130 for connecting the partial storage portion 120 and the remaining portion of the storage space is arranged in the liquid receptor 15a. An opening 132 of the communication passage 130 on the remaining portion side is arranged above the bottom surface of the liquid receptor 15a. As a result, the opening 132 of the communication passage 130 on the remaining portion side is located in a position higher than an opening 134 on the partial storage portion side. With such an arrangement, the warm water heated in the partial storage portion 120 can be retained in the partial storage portion 120. If the water level in the partial storage portion 120 falls, the liquid 2 on the remaining portion side moves to the partial storage portion 120 side via the communication passage 130. Since the opening 132 of the communication passage 130 on the remaining portion side is arranged near the prescribed water level, only the warm water heated on the remaining portion side and residing at the water surface can be exclusively transferred to the side of the partial storage portion 120.

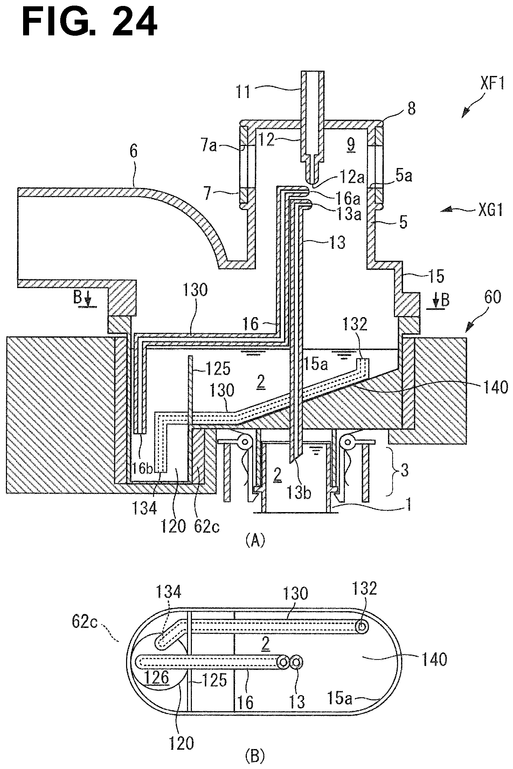

Moreover, as shown in an application example of FIG. 24, a slope 140 may be arranged on at least a part of the bottom surface of the liquid receptor 15a. The presence of this slope 140 can change the depth (water depth) of the liquid receptor 15a, so that cold water can move to greater depths along the slope 140 and warm water can reside in shallower depths. Consequently, if the opening 132 of the communication passage 130 on the remaining portion side is arranged in the area of shallow depths, warm water can be more efficiently transferred to the partial storage portion 120. Note that the partition wall 125, a lid, or the like is preferably arranged to prevent the cold water moving to greater depths along the slope 140 from entering the partial storage portion 120. It will be understood that such a slope 140 may also be formed in the configuration described in the first embodiment so that the receptor-side aerosol forming member 16 can preferentially suck the liquid 2 in the area of shallow depths to heat the mixed gas efficiently.

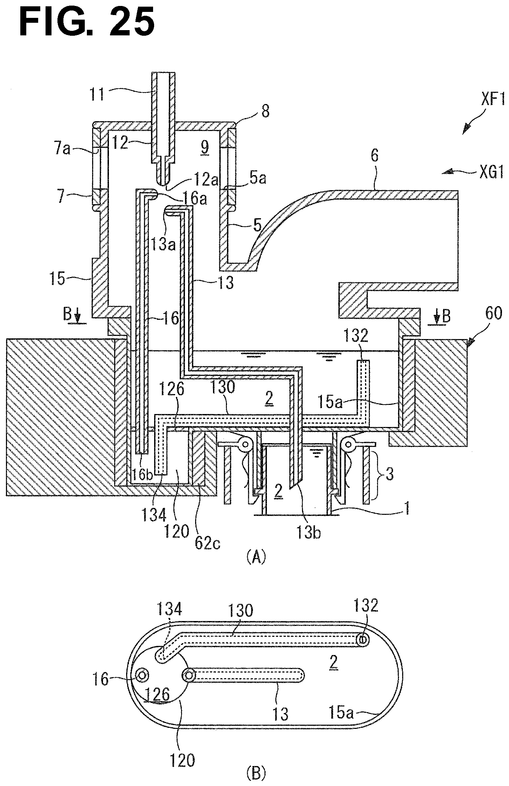

The foregoing first to third embodiments have described examples of a structure in which the orifice 12a of the nozzle member 12 is arranged vertically above the adaptor-side connection portion 13. However, the present invention is not limited thereto. As in another application example shown in FIG. 25, the orifice 12a of the nozzle member 12 may be arranged vertically above the partial storage portion 120. In such a case, a lid can be arranged on the partial storage portion 120 to suppress the rippling effect of the water surface.

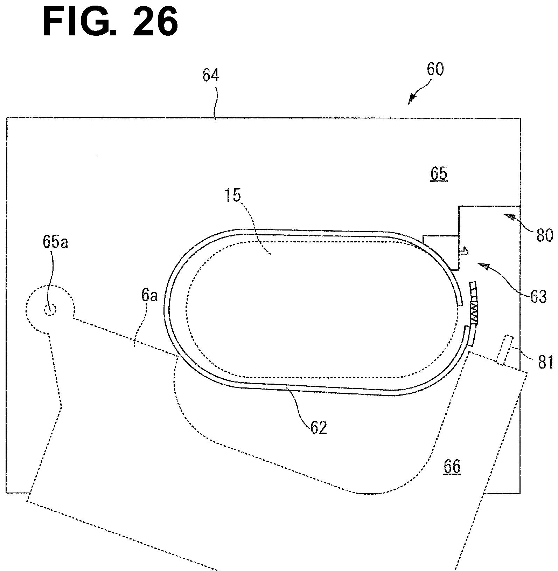

In the foregoing first to third embodiments, the heater device 60 is described to be configured so that the liquid receptor 15a is sandwiched between the pair of plate heaters 62a and 62b. However, the present invention is not limited thereto. For example, as in an application example of the heater device 60 shown in FIG. 26, one flexible plate heater 62 may be automatically or manually wound therearound, and its ends can be fixed by a fixing jig 63 to fix the heater in close contact with the liquid receptor. Even in such a case, the heater 62 is preferably covered with the main case 65 and the swing case 66 to prevent the operator from getting burned etc. While the heater device 60 is described to heat the side surface of the liquid receptor, the heater device 60 may be configured to heat the liquid receptor from the bottom surface or top surface side.

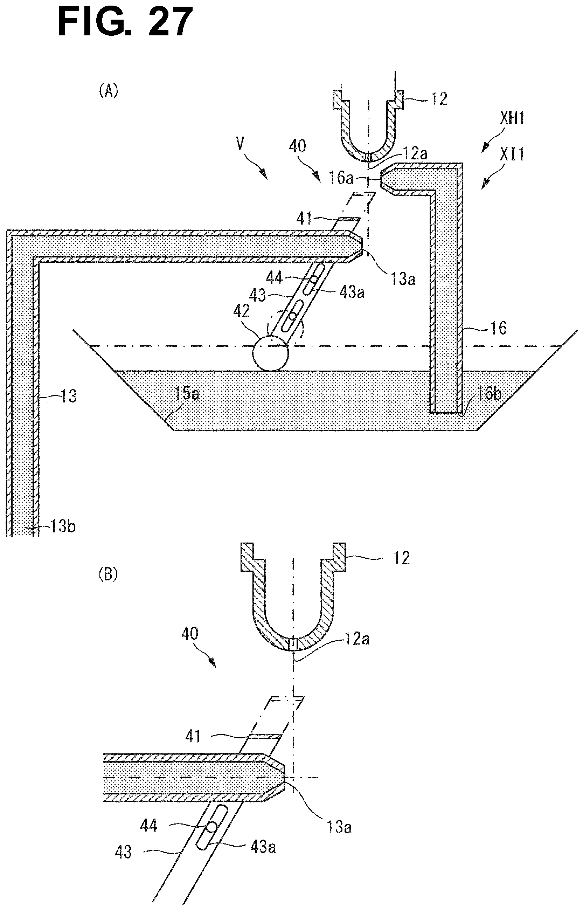

[Fourth Embodiment] Next, a configuration of a nebulizer XH1 according to a fourth embodiment will be described with reference to FIG. 27. FIG. 27(A) is a conceptual diagram for describing a structure of a valve 40 of a liquid surface control mechanism V included in a nebulizer adaptor XI1 which constitutes the nebulizer XH1. FIG. 27(B) is an enlarged view showing essential parts of the structure shown in FIG. 27(A).

The nebulizer (humidifying device) XH1 shown in FIGs. 27(A) and 27(B) includes the nebulizer adaptor XI1, and a heater device and a water bottle (container) which are not shown in the diagrams.

The nebulizer adaptor XI1 includes the valve 40 which opens and closes vertically above the ejection port 13a of the container-side aerosol forming member 13 to interfere with the impingement of the oxygen gas jetted from the orifice 12a on the ejection port 13a. The valve 40 is of an "oblique slide system" in which the oxygen gas jetted from the orifice 12a is cut off. Specifically, the valve 40 includes a cutoff member 41, a floating member 42, and a connection member 43.

The cutoff member 41 makes an oblique sliding movement between a cutoff position (position shown by the dashed-dotted lines in FIG. 27(A)) in which to cut off the ejection port 13a from the orifice 12a and a retracted position (position shown by the solid lines in FIG. 27(A)) in which to be retracted from the cutoff position. If the cutoff member 41 is located in the cutoff position, the cutoff member 41 cuts off the ejection port 13a from the orifice 12a to stop the function of sucking up the liquid 2 by the container-side aerosol forming member 13. If the cutoff member 41 is located in the retracted position, the cutoff member 41 cancels the cutting off of the ejection port 13a from the orifice 12a to restore the function of sucking up the liquid 2 by the container-side aerosol forming member 13.

The floating member 42 floats on the liquid surface of the liquid 2 stored in the liquid receptor 15a, and floats up and down according to a rise or fall of the liquid surface. Guide grooves 43a to be guided by guide pins 44 formed on the housing (not shown) of the nebulizer adaptor XI1 are formed in the connection member 43. This connection member 43 connects the floating member 42 to the cutoff member 41, and causes the cutoff member 41 to make a sliding movement with the floating of the floating member 42.

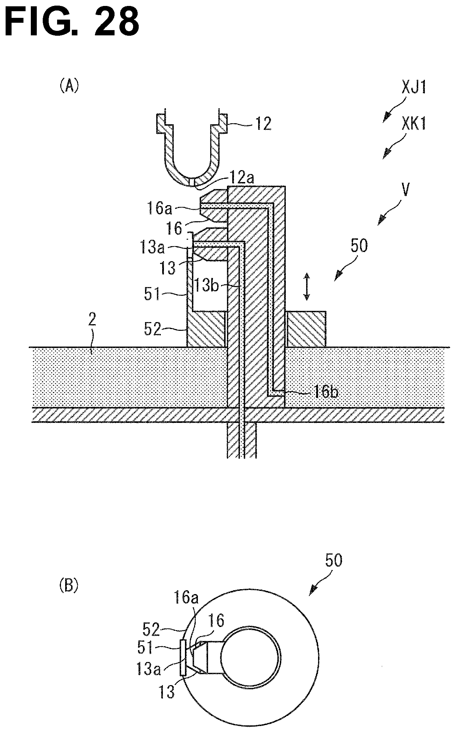

[Fifth Embodiment] Next, a configuration of a nebulizer XJ1 according to a fifth embodiment will be described with reference to FIG. 28.

The nebulizer (humidifying device) XJ1 shown in FIGs. 28(A) and 28(B) includes a nebulizer adaptor XK1, and a heater device and a water bottle (container) which are not shown in the diagrams.

The nebulizer adaptor XK1 includes, as a liquid surface control mechanism V, a valve 50 which opens and closes the ejection port 13a of the container-side aerosol forming member 13. The valve 50 is of a "vertical slide system" configured to block the ejection port 13a. Specifically, the valve 50 includes a cutoff plate 51 and a float ring 52 which is a floating member.

The cutoff plate 51 is configured integrally with the float ring 52 and makes a sliding movement with the float ring 52. The cutoff plate 51 thus makes a vertical sliding movement between a cutoff position (position shown by the dashed-dotted lines in FIG. 28(A)) in which to block the ejection port 13a and a retracted position (position shown by the solid lines in FIG. 28(A)) in which to be retracted from the cutoff position. If the cutoff plate 51 is located in the cutoff position, the cutoff plate 51 blocks the ejection port 13a to stop the function of sucking up the liquid 2 by the container-side aerosol forming member 13. If the cutoff plate 51 is located in the retracted position, the cutoff plate 51 cancels the blocking of the ejection port 13a to restore the function of sucking up the liquid 2 by the container-side aerosol forming member 13.

The float ring 52 is slidably fitted onto the container-side aerosol forming member 13 and the receptor-side aerosol forming member 16 which are integrally configured. This float ring 52 floats on the liquid surface of the liquid 2 stored in the liquid receptor 15a, and floats up and down according to a rise or fall of the liquid surface. The float ring 52 thereby causes the cutoff plate 51 to make a vertical sliding movement.

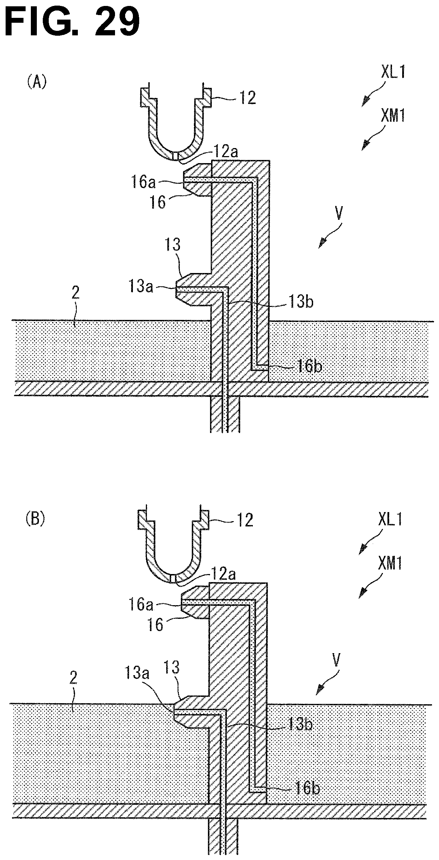

[Sixth Embodiment] Next, a configuration of a nebulizer XL1 according to a sixth embodiment will be described with reference to FIG. 29.

The nebulizer (humidifying device) XL1 shown in FIGs. 29(A) and 29(B) includes a nebulizer adaptor XM1, and a heater device and a water bottle (container) which are not shown in the diagrams.

In the nebulizer adaptor XM1, the ejection port 13a of the container-side aerosol forming member 13 is arranged, as a liquid surface control mechanism V, at approximately the same height as the prescribed liquid level. That is, the nebulizer adaptor XM1 is of a "submersion system" in which the ejection port 13a is blocked by the liquid surface of the liquid 2.

In the state shown in FIG. 29(A), the water surface of the liquid 2 is lower than the ejection port 13a. The exposed ejection port 13a enables the sucking up of the liquid 2 in the water bottle. On the other hand, in the state where the water level of the liquid 2 rises and the ejection port 13a is submerged as in FIG. 29(B), the function of sucking up the liquid 2 by the container-side aerosol forming member 13 stops. If the liquid 2 is consumed to become an aerosol and the liquid surface falls, the state returns to that of FIG. 29(A) again. The ejection port 13a is exposed and the function of sucking up the liquid 2 by the container-side aerosol forming member 13 is restored.

In the present embodiment, the ejection portion 13a of the container-side aerosol forming member 13 is located closer to the water surface side. Since the ejection port 13a is farther from the orifice 12a, the negative pressure may become insufficient. In such a case, the flow of the oxygen gas may be branched to form a second orifice aside from the orifice 12a corresponding to the ejection port 16a of the receptor-side aerosol forming member 16. The second orifice may be located close to the ejection port 13a of the container-side aerosol forming member 13 which is arranged near the prescribed liquid level.

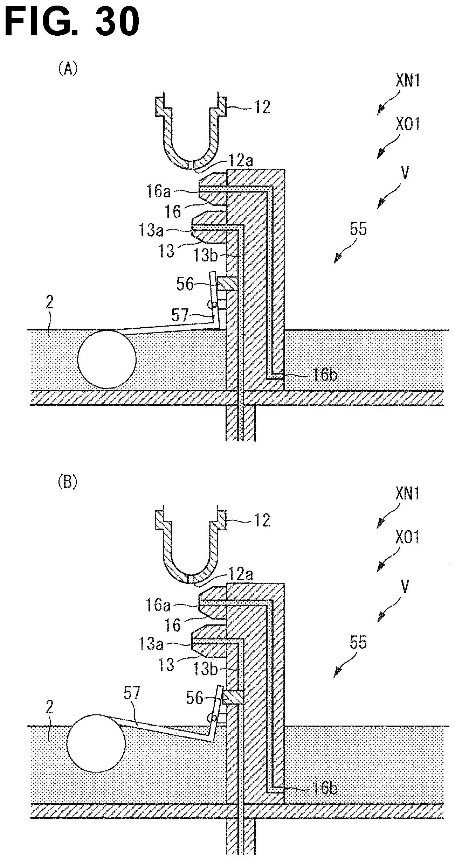

[Seventh Embodiment] Next, a configuration of a nebulizer XN1 according to a seventh embodiment will be described with reference to FIG. 30.

The nebulizer (humidifying device) XN1 shown in FIGs. 30(A) and 30(B) includes a nebulizer adaptor XO1, and a heater device and a water bottle (container) which are not shown in the diagrams.

The nebulizer adaptor XO1 includes, as a liquid surface control mechanism V, a valve 56 which is arranged on the way of the transfer channel 13b of the container-side aerosol forming member 13, and a driving member 57 configured to swing to turn ON/OFF the valve 56 by using buoyancy according to a rise or fall of the liquid 2. The valve 56 moves to and from the transfer channel 13b to close and open the flow path of the transfer channel 13b. As shown in FIG. 30(A), if the water level of the liquid 2 is low, the valve 56 opens the transfer channel 13b so that the container-side aerosol forming member 13 can suck up the liquid 2. On the other hand, as shown in FIG. 30(B), if the water level of the liquid 2 reaches the prescribed water level, the driving member 57 swings and presses in the valve 56 to close the transfer channel 13b. As a result, the function of sucking up the liquid 2 by the container-side aerosol forming member 13 stops.

In the foregoing first to seventh embodiments, the liquid surface control mechanism V is described to switch the suction function of the container-side aerosol forming member 13 by using the buoyancy from the liquid 2 or the liquid itself. However, the present invention is not limited thereto. For example, the liquid surface (water level) of the liquid 2 may be electrically measured, and the measurement result may be used to switch ON/OFF the flow of the transfer channel 13b with an electrical valve. If the liquid is sucked up from the water bottle by an electrical pump, the pump function may be switched ON/OFF to control the liquid surface by using the measurement result of the liquid surface.

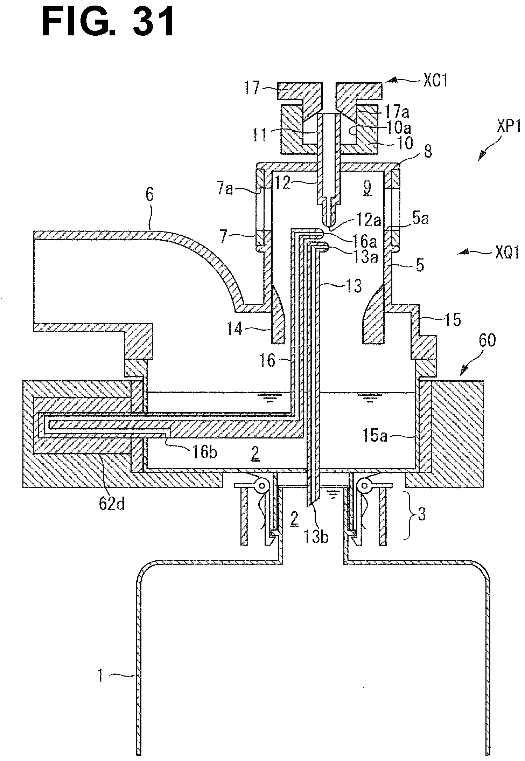

[Eighth Embodiment] Next, a configuration of a nebulizer XP1 according to an eighth embodiment will be described with reference to FIG. 31. Here, characteristic parts of the nebulizer XP1 will be mainly described. A configuration and operation similar to or the same as those of the foregoing first embodiment will be denoted by the same reference numerals in the drawings, and a description thereof may be omitted as appropriate.

This nebulizer (humidifying device) XP1 includes a nebulizer adaptor XQ1, the heater device 60, and the water bottle (container) 1.

In this nebulizer adaptor XQ1, a part of the liquid channel of the receptor-side aerosol forming member 16 is arranged outside the liquid receptor 15a or in a wall surface of the liquid receptor 15a and heated by the heater device 60. The heater device 60 includes a liquid channel heating heater 62d, and indirectly heats the liquid 2 passing through the receptor-side aerosol forming member 16 from outside. As a result, the liquid 2 lying between the liquid suction port 16b and the ejection port 16a of the receptor-side aerosol forming member 16 can be efficiently heated. It will be understood that the liquid 2 in the liquid receptor 15a is also preferably heated.

In the foregoing first to eighth embodiments, the heater device 6 is described to heat only the liquid 2 that is once stored in the liquid receptor 15a. In addition, the liquid 2 that is in the process of being sucked up from the water bottle 1 by the container-side aerosol forming member 13 may be heated.

In the foregoing first to eighth embodiments, a mechanism for sucking up and transferring the liquid 2 from the water bottle 1 to the liquid receptor 15a by using the container-side aerosol forming member 13 is described as the liquid transfer mechanism. However, other means such as a pump mechanism may be used to transfer the liquid 2 from the water bottle 1 to the liquid receptor 15a.

### [Status Determination Processing of Nebulizer Adaptor]

Next, an example of a case where status determination processing of the nebulizer adaptor XB1 is added to the basic control flow of the heater device 60 shown in FIG. 14 will be described with reference to the nebulizer XA1 according to the first embodiment. Differences from the first embodiment will be described here, and a description of the same or similar parts will be omitted.

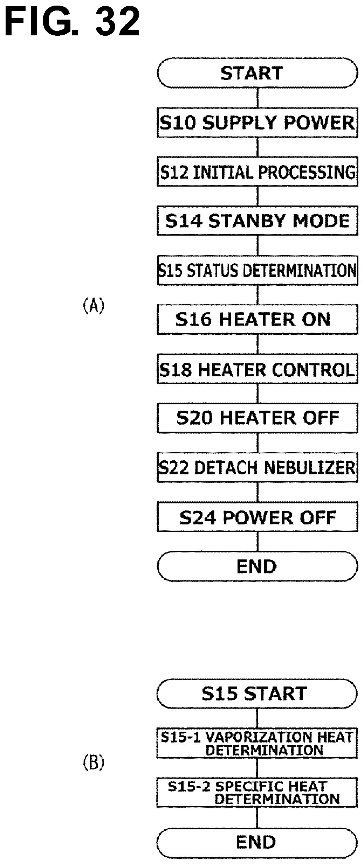

FIG. 32(A) shows a basic control flow of the heater device 60, in which the status determination processing of the nebulizer adaptor XB1 is added as step S15 between the standby mode of step S14 and the heater ON of step S16. As shown in FIG. 32(B), the status determination processing of step S15 includes vaporization heat determination processing of step S15-1 and specific heat determination processing of step S15-2.

In the vaporization heat determination processing of step S15-1, temperature variations of the gas temperature sensor 90 and temperature variations of the heater (temperature) sensors 91a and 91b of the plate heaters 62a and 62b are measured with the output of the plate heaters 62a and 62b OFF. When using this nebulizer XA1, the operator is supposed to have an aerosol ejected from the ejection port 16a of the receptor-side aerosol forming member 16 before turning ON the plate heaters 62a and 62b. If the aerosol is ejected from the ejection port 16a, the temperature of the liquid 2 accumulated in the liquid receptor 15a decreases due to the vaporization heat, and at the same time the temperature of the mixture of the air, the oxygen gas, and the aerosol decreases. In the vaporization heat determination processing, a temperature drop of the liquid 2 is thus detected by the heater (temperature) sensors 91a and 91b and a temperature drop of the mixture is detected by the gas temperature sensor 90 to automatically determine that the aerosol is being ejected before the heater is turned ON. If there is no drop in temperature, one of the following is determined to be the case: (1) the nebulizer adaptor XB1 itself is not attached; (2) the nebulizer adaptor XB1 is attached and the liquid receptor 15a contains no liquid 2; and (3) the liquid 2 is accumulated in the liquid receptor 15a but an aerosol is not ejected from the ejection port 16a for reasons such as that no oxygen gas is jetted from the orifice 12a. In any case, control is performed to be an error and not proceed to the heater ON of step S16.

In the specific heat determination of step S15-2, the output of the plate heaters 62a and 62b is turned ON, and the specific heat (or heat capacity) of the target object (the nebulizer adaptor XB1 and the liquid 2 inside) in contact with the plate heater 62a and 62b is determined. Specifically, there are the following two methods for specific heat determination, (technique A) and (technique B).

(Technique A) Control the output of the plate heaters 62a and 62b to be constant, and detect the speed of the temperature increase of the heater (temperature) sensors 91a and 91b. If the temperature increase is slow, the specific heat or heat capacity of the target object is determined to be high. If the temperature increase is fast, the specific heat or heat capacity of the target object is determined to be low.

Consequently, (1) if the nebulizer adaptor XB1 itself is not attached, the speed of the temperature increase is the highest. (2) If the nebulizer adaptor XB1 is attached and the liquid receptor 15a contains no liquid 2, the speed is lower than in (1). (3) If the liquid 2 is accumulated in the liquid receptor 15 but an aerosol is not ejected from the ejection port 16a for reasons such as that no oxygen is jetted from the orifice 12a, the speed is even lower than in (2). (4) If the liquid 2 is accumulated in the liquid receptor 15a and an aerosol is ejected from the ejection port 16a, the speed is the lowest. Therefore, differences between the specific heats of (1) to (4) can be detected to determine, for example, that the status is normal in the case of (3) or (4). In such a case, the processing may be allowed to proceed to the next step S16. If the target object has a low specific heat, the temperature of the plate heaters 62a and 62b can increase sharply with an adverse effect on the surrounding members. Then, the following procedure B is a practical alternative to take.

(Technique B) Perform feedback control on the temperature of the plate heaters 62a and 62b so that the temperature stabilizes in a temperature range of higher than the ambient temperature and lower than in normal heating (for example, so as not to cause a burn injury). Detect the output of the plate heater 62a and 62b when the temperature is stabilized. If the output is high, the specific heat or the heat capacity of the target object is determined to be high. If the output of the plate heaters 62a and 62b is low, the specific heat or the heat capacity is determined to be low.

(1) If the nebulizer adaptor XB1 itself is not attached, the specific heat or heat capacity of the target object is the lowest. (2) If the nebulizer adaptor XB1 is attached and the liquid receptor 15a contains no liquid 2, the specific heat or heat capacity is higher than in (1). (3) If the liquid 2 is accumulated in the liquid receptor 15a but an aerosol is not ejected from the ejection port 16a for reasons such as that no oxygen gas is jetted from the orifice 12a, the specific heat or heat capacity is higher than in (2). (4) If the liquid 2 is accumulated in the liquid receptor 15a and an aerosol is ejected from the ejection port 16a, the specific heat or heat capacitance is the highest. Differences in the specific heats of (1) to (4) can thus be detected to determine various states. For example, in the case of (3) or (4), the status may be determined to be normal and the processing may be allowed to proceed to the next step S16. Various determination thresholds can be used to determine the status of the nebulizer adaptor in details.

Consequently, the presence of the status determination processing of step S15 can suppress installation errors by the operator and so-called no-water burning by the plate heaters 62a and 62b.

The status determination processing of step S15 has been described to include both the vaporization heat determination of step S15-1 and the specific heat determination of step S15-2. However, the present invention is not limited thereto, and either one of the determinations may be used alone. The timing to perform the status determination processing may also be changed as appropriate.
For example, the specific heat determination of step S15-2 is also preferably performed in the initial heating processing of step S34 in FIG. 15 when the plate heaters 62a and 62b are set to the first output to wait for a certain time for temperature stabilization.

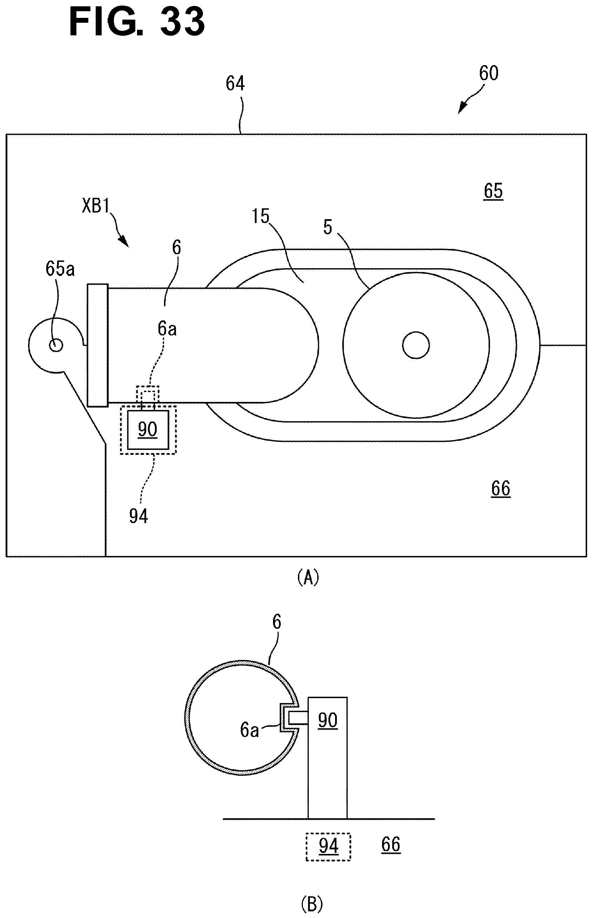

[Temperature Correction of Gas Temperature Sensor] Next, an application procedure for accurately estimating the temperature of the mixed gas with respect to the temperature measured by the gas temperature sensor 90 in the nebulizer XA1 of the first embodiment will be illustrated. As shown in FIG. 33, a heat transfer measurement temperature sensor 94 is additionally arranged on a side closer to the swing case 66 than the gas temperature sensor 90 is, or inside the swing case 66. This heat transfer measurement temperature sensor 94 is located in a position unaffected by a change in the temperature of the mixed gas passing through the horizontal projection portion 6, the position being on the way of a heat transfer path through which the heat of the plate heaters 62a and 62b is transferred to the gas temperature sensor 90. The heat of the plate heaters 62a and 62b is transferred to the gas temperature sensor 90 via the swing case 66 and causes measurement errors of the gas temperature sensor 90. The present inventors have revealed that the temperature of the mixed gas can be accurately estimated by detecting how much heat is transferred from the plate heaters 62a and 62b to the gas temperature sensor 90 by using the heat transfer measurement temperature sensor 94, and subtracting a predetermined proportion of the temperature value of the heat transfer measurement temperature sensor 94 from the temperature detection result of the gas temperature sensor 90. This predetermined proportion may be changed according to the installation position of the heat transfer measurement temperature sensor 94, the material of the swing case, the outside air temperature, the spray flow rate, etc. As a result, the detection accuracy of the temperature of the mixed gas can be improved.

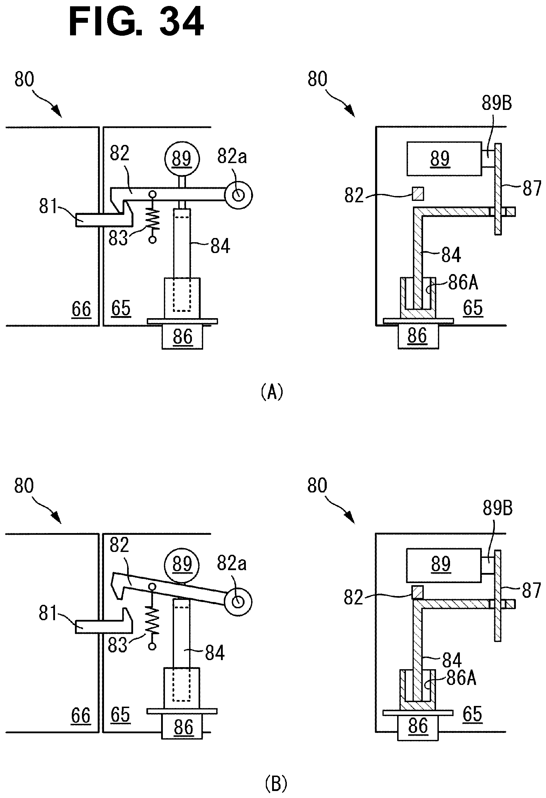

[Configuration and Control of Manual Lock mechanism] Next, an example of an application configuration of the lock mechanism 80 in the nebulizer XA1 of the first embodiment will be described. As shown in FIG. 34(A), the lock mechanism 80 includes a fixed engagement portion 81 which is arranged on a side of the swing case 66, a swing engagement portion 82 which is arranged on a side of the main case 65 to be swingable about a swing shaft 82a, a locking spring 83 configured to bias the swing engagement portion 82 to a lock side, an operation arm 84 configured to forcefully swing the swing engagement portion 82 in an opening direction, an opening/closing button 86 configured to manually move the operation arm 84 up and down, a locking plunger 89 configured to switch between engagement and disengagement of the operation arm 84 and the swing engagement portion 82, and a control rod 87 which is moved to reciprocate by the locking plunger 89. The fixed engagement portion 81 and the swing engagement portion 82 are engaged with each other at their ends, whereby the swing case 66 and the main case 75 are maintained in a closed state. The opening/closing button 86 includes a cylindrical portion 86A inside the main case 65. A lower end of the operation arm 84 is accommodated in the cylindrical portion 86A. The internal space of the cylindrical portion 86A is set to be greater than the operation arm 84 so that the lower end of the operation arm 84 can swing inside the cylindrical portion 86A as will be described later.

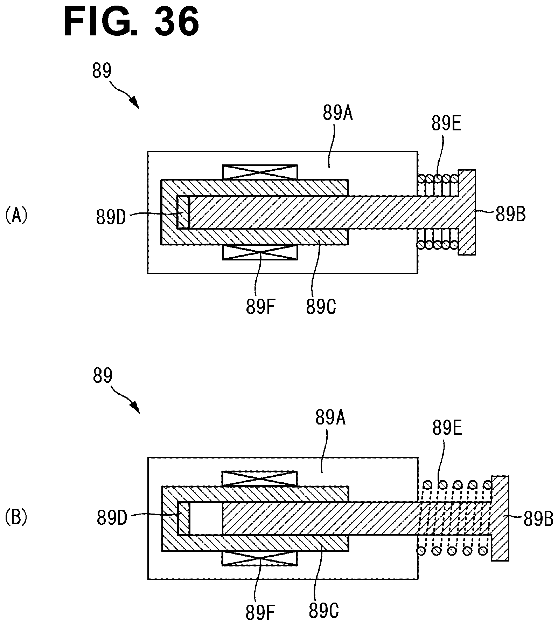

The locking plunger 89 is of so-called latch type. The locking plunger 89 maintains an immediately previous position when its power supply is turned OFF. Specifically, as shown in FIGs. 36(A) and 36(B), the locking plunger 89 includes a main body portion 89A of cylindrical shape, a sliding shaft 89B which is arranged to be axially movable by the main body portion 89A, a yoke portion 89C which is arranged in the main body portion 89A, an exciting coil 89F which is arranged adjacent to the yoke portion 89C and configured to generate magnetic force for driving the sliding shaft 89B, a fixed magnet 89D configured to generate attractive force for holding the sliding shaft 89B in a position where the sliding shaft 89B is in a contracted state, and a coil spring 89E configured to bias the sliding shaft 89B in a direction toward an extended state. The yoke portion 89C has a U shape to surround an inner end of the sliding shaft 89B. The fixed magnet 89D is arranged at the bottom of the yoke portion 89C.

As shown in FIG. 36(A), if the exciting coil 89F is energized, the direction of the magnetic lines of force occurring along the yoke portion 89C and that of the magnetic lines of force of the fixed magnet 89D coincide to increase the attractive force near the fixed magnet 89D. The sliding shaft 89B is magnetically attracted against the biasing force of the coil spring 89E, and the inner end of the sliding shaft 89B approaches the fixed magnet 89D. Consequently, if the energization of the exciting coil 89F is turned off in such a contracted state, the attractive force between the sliding shaft 89B and the fixed magnet 89D lying close to each other exceeds the biasing force of the coil spring 89E, whereby the contracted state is maintained.

On the other hand, as shown in FIG. 36(B), if the exciting coil 89F is energized in a direction opposite from in the contracted state, the magnetic lines of force occurring along the yoke portion 89C and the magnetic lines of force of the fixed magnet 89D become opposite in direction and cancel out to reduce the attractive force near the fixed magnet 89D. As a result, the biasing force of the coil spring 89E becomes dominant to extend the sliding shaft 89B. If the energization of the exciting coil 89F is turned OFF in such an extended state, the extended state of the sliding shaft 89B is maintained by the biasing force of the coil spring 89E.

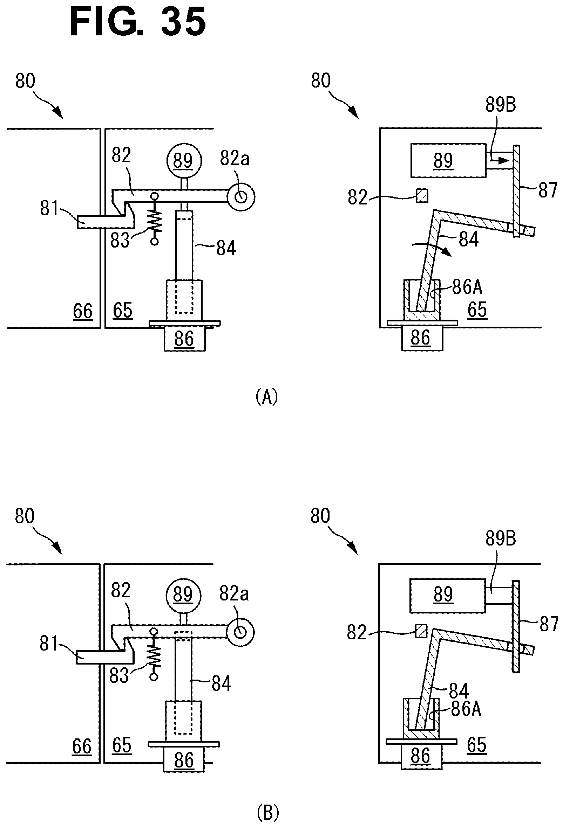

Return to FIG. 34(A). The sliding shaft 89B of the locking plunger 89 is connected with the control rod 87. The control rod 87 is engaged with the operation arm 84. Consequently, if the control rod 87 is displaced by the locking plunger 89, the operation arm 84 can swing or move to switch between a state in which the operation arm 84 and the swing engagement portion 82 can be engaged with each other (unlockable state/see FIG. 34) and a state in which the operation arm 84 and the swing engagement portion 82 are not able to be engaged with each other (non-unlockable state/see FIG. 35).

The opening/closing button 86 is arranged on the bottom surface side of the main case 65. If the opening/closing button 86 is pressed in toward the top surface side, as shown in FIG. 34(B), the operation arm 84 in the main case 65 rises to swing the swing engagement portion 82 to the open side against the biasing force of the locking spring 83. The swing engagement portion 82 is disengaged from the fixed engagement portion 81 to unlock the lock mechanism 80. Since the opening/closing button 86 is arranged on the bottom surface side, the operator presses the opening/closing button 86 while holding the main case 65 in hand. This prevents the heater device from falling even if the main case 65 and the swing case 66 are suddenly opened.

If the locking plunger 89 is in the extended state, as shown in FIG. 35(A), the operation arm 84 is swung via the control rod 87 with the lower end as the fulcrum. As a result, the upper end of the operation arm 84 is retracted from the swing engagement portion 82. If the operator presses in the opening/closing button 86 toward the top surface side in such a state, as shown in FIG. 35(B), the lifted operation arm 84 passes by the swing engagement portion 82. Since the engagement portion 82 is not swung, the lock mechanism 80 will not be unlocked.

As can be seen from the foregoing configuration, according to such a lock mechanism 80, the unlockable state and the non-unlockable state can be freely switched by bringing the locking plunger 89 into the extended state or the contracted state.

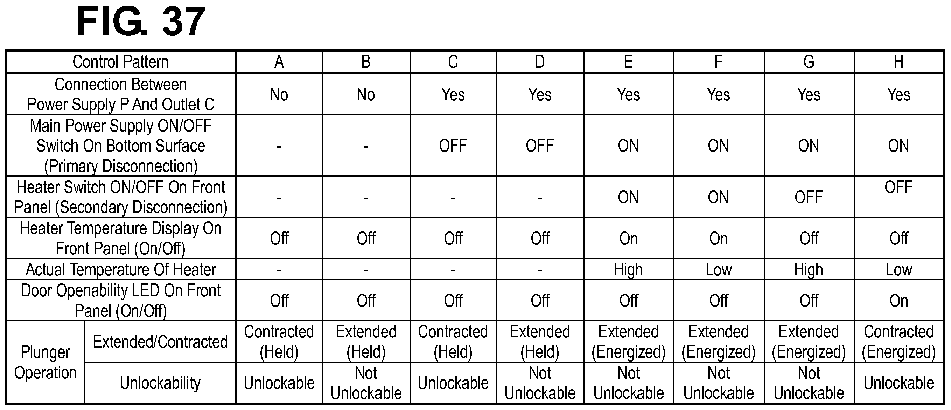

FIG. 37 shows control patterns of the locking plunger 89. Here, the heater device of the nebulizer XA1 is assumed to have a main power switch on the bottom surface side.

Control pattern E represents a case where the power supply P and the outlet C are connected to each other, the main power switch is ON, the heater switch is ON, and the heater is at high temperature (higher than a predetermined threshold). In such a case, the locking plunger 89 is controlled to the extended state of FIG. 35 and the lock mechanism 80 becomes unable to be unlocked. The reason is that the user might get burned by the heater.

Control pattern F represents a case where the heater is at low temperature (lower than the predetermined threshold) as compared to control pattern E. Even in such a case, the locking plunger 89 is controlled to the extended state of FIG. 35 and the lock mechanism 80 is unable to be unlocked. The reason is that the heater is, though at low temperature, being heated and the operator might get burned by a subsequent temperature increase of the heater.

Control pattern G represents a case where the heater switch is OFF as compared to control pattern E. Even if the heater is OFF, the heater temperature is high. The locking plunger 89 is therefore also controlled to the extended state of FIG. 35 and the lock mechanism 80 is unable to be unlocked. The reason is that cooling is determined to be insufficient.

Control pattern H represents a case where the heater switch is OFF and the heater is at low temperature as compared to control pattern E. Only in such a pattern, the locking plunger 89 is controlled to the contracted state of FIG. 34 and the lock mechanism 80 becomes able to be unlocked.

Control pattern C represents a mode where the main power supply is turned OFF in the state of control pattern H. As described previously, the locking plunger 89 of the lock mechanism 80 is held in its immediately previous state even if the energization is turned OFF. The locking plunger 89 is thus held in the contracted state of FIG. 34 and the lock mechanism 80 is able to be unlocked.

Control pattern D represents a mode where the main power supply is turned OFF in the state of control pattern E, F, or G. The locking plunger 89 of the lock mechanism 80 is held in its immediately previous state even if the energization is turned OFF. The locking plunger 89 is thus held in the extended state of FIG. 35 and the lock mechanism 80 is unable to be unlocked. To enable the unlocking of the lock mechanism 80 in such a state, the main power supply can be once turned ON to enter control pattern H when the heater is at sufficiently low temperature.

Control pattern A represents a mode where the power supply P and the outlet C are disconnected in the state of control pattern C or H. The locking plunger 89 of the lock mechanism 80 is held in its immediately previous state even if the energization is turned OFF. The locking plunger 89 is thus held in the contracted state of FIG. 34 and the lock mechanism 80 is able to be unlocked.

Control pattern B represents a mode where the power supply P and the outlet C are disconnected in the state of control pattern D, E, F, or G. The locking plunger 89 of the lock mechanism 80 is held in its immediately previous state even if the energization is turned OFF. The locking plunger 89 is thus held in the extended state of FIG. 35 and the lock mechanism 80 is unable to be unlocked. To enable the unlocking of the lock mechanism 80 in such a state, the main power supply can be turned ON to enter control pattern H when the heater is at sufficiently low temperature.

The present invention is not limited to the foregoing embodiments, and various modifications may be made without departing from the gist and technical concept thereof. The configurations of the embodiments and modifications may be applied to other embodiments and modifications as far as possible.

More specifically, in the foregoing embodiments, the positions, sizes (dimensions), shapes, materials, directions, numbers, and the like of the components may be changed as appropriate.

### Reference Signs List

XA1, XD1, XF1, XH1, XJ1, XL1, XN1, XP1 nebulizer (humidifying device)
XB1, XE1, XG1, XI1, XK1, Xm1, XO1, XQ1 nebulizer adaptor (adaptor)
XC1 oxygen flowmeter
1 water bottle (container)
2 liquid such as sterile water
3 adaptor-side connection portion
5 erected projection portion
6 horizontal projection portion (sending unit)
7 adjustment dial
8 top plate
10 nut
11 terminal
12 nozzle member
12a orifice
13 container-side aerosol forming member
14 diffuser
15 housing of nebulizer adaptor
15a liquid receptor
16 receptor-side aerosol forming member
17 outlet member of oxygen flowmeter
60 heater device
62a, 62b plate heater
64 case
65 main case
66 swing case
70 operation panel
80 lock mechanism

## Claims

1. A humidifying device comprising:
a liquid receptor configured to temporarily store a liquid;
a liquid transfer mechanism configured to transfer a liquid for humidification from a container containing the liquid to the liquid receptor;
a receptor-side aerosol forming member configured to suck the liquid of the liquid receptor from a liquid suction port by a negative pressure produced by a gas jetted from a gas jetting part of a nozzle member, and form an aerosol of the sucked liquid with a receptor-side aerosol forming nozzle; and
a heating mechanism configured to heat at least part of the liquid of the liquid receptor or the liquid in the receptor-side aerosol forming member.

2. The humidifying device according to claim 1, wherein
the heating mechanism is arranged around the liquid receptor or the receptor-side aerosol forming member and configured to indirectly heat the liquid of the liquid receptor or the liquid in the receptor-side aerosol forming member from outside the member.

3. The humidifying device according to claim 1 or 2, wherein
the heating mechanism is detachably arranged on the liquid receptor or the receptor-side aerosol forming member.

4. The humidifying device according to any one of claims 1 to 3, wherein:
a connection portion configured to connect to the container is formed under the liquid receptor; and
the heating mechanism is arranged on the liquid receptor or the receptor-side aerosol forming member so that the connection portion is exposed.

5. The humidifying device according to any one of claims 1 to 4, wherein
the heating mechanism includes a pair of heater members that open and close horizontally, and the heater members are arranged to sandwich the liquid receptor from both side surfaces.

6. The humidifying device according to any one of claims 1 to 5, wherein
the receptor-side aerosol forming member has the liquid suction port in a position horizontally shifted from a position vertically directly below the receptor-side aerosol forming nozzle.

7. The humidifying device according to any one of claims 1 to 6, wherein
the liquid suction port of the receptor-side aerosol forming member is arranged near a liquid surface of the liquid of the liquid receptor.

8. The humidifying device according to any one of claims 1 to 7, wherein
a restriction member configured to restrict impingement of the gas jetted from the gas jetting part of the nozzle member on the liquid surface is arranged.

9. The humidifying device according to any one of claims 1 to 8, wherein:
the liquid receptor includes a partial storage portion configured to section a part of a storage space storing the liquid;
the heating mechanism heats at least the liquid of the partial storage portion; and
the liquid suction port of the receptor-side aerosol forming member is arranged to suck the liquid of the partial storage portion.

10. The humidifying device according to claim 9, wherein
a partition wall is erected in the storage space of the liquid receptor, and the partial storage portion and a remaining portion are sectioned by the partition wall.

11. The humidifying device according to claim 9 or 10, wherein
the liquid receptor includes a communication passage configured to connect the partial storage portion and a remaining portion of the storage space.

12. The humidifying device according to claim 11, wherein
an opening of the communication passage on a side of the remaining portion is arranged in a position higher than an opening on a side of the partial storage portion.

13. The humidifying device according to any one of claims 1 to 12, wherein
a slope is formed on at least a part of a bottom surface of the liquid receptor.

14. The humidifying device according to any one of claims 1 to 13, wherein
the liquid transfer mechanism includes a transfer channel configured to suck the liquid for humidification from the container containing the liquid and discharge the liquid to the liquid receptor by using the negative pressure produced by the gas jetted from the gas jetting part of the nozzle member.

15. The humidifying device according to claim 14, wherein
a discharge port of the liquid of the transfer channel includes a container-side aerosol forming nozzle configured to form an aerosol of the liquid sucked from the container.

16. The humidifying device according to claim 14 or 15, comprising:
a cutoff member configured to move between a cutoff position in which to cut off the discharge port of the liquid of the transfer channel from the gas jetting part and a retracted position in which to be retracted from the cutoff position;
a floating member configured to float up and down according to a rise or fall of the liquid surface of the liquid stored in the liquid receptor; and
a connection member configured to connect the floating member to the cutoff member and cause the cutoff member to move with floating of the floating member.

17. The humidifying device according to any one of claims 1 to 16, comprising:
a determination device configured to determine lowering of the liquid surface of the liquid of the liquid receptor; and
notification means configured to issue a notification that the container containing the liquid becomes empty on a basis of a result of determination of the determination device

18. The humidifying device according to claim 17, comprising a heater temperature sensor configured to measure a temperature of a heater configured to heat the liquid with the heating mechanism, wherein
the determination device determines the lowering of the liquid surface of the liquid in the liquid receptor on a basis of a measurement value of the heater temperature sensor.

19. The humidifying device according to claim 18, wherein
the determination device determines that the liquid surface of the liquid in the liquid receptor has lowered if an output of the heater is not increased and the temperature of the heater increases.

20. The humidifying device according to claim 18, further comprising a gas temperature sensor configured to measure a temperature of a gas humidified by the aerosol, wherein
the determination device determines that the liquid surface of the liquid in the liquid receptor has lowered if the temperature of the humidified gas deviates from a set temperature and the temperature of the heater increases.

21. The humidifying device according to any one of claims 1 to 20, comprising an adaptor that is detachably attached to the container, the adaptor including at least the receptor-side aerosol forming member, the liquid receptor, and the liquid transfer mechanism.

22. The humidifying device according to any one of claims 1 to 21, the humidifying device being configured to humidify and send the gas containing oxygen to a patient, the humidifying device comprising a sending unit configured to send out a mixture of the gas and the aerosol.

23. The humidifying device according to any one of claims 1 to 22, comprising the container.
